# EUROPEAN PATENT APPLICATION

(11) **EP 3 109 319 A1**
(43) Date of publication of application: **28.12.2016**
(21) Application number: 16182020.4
(22) Date of filing: 02.10.2003
(51) Int. Cl.: C12N 9/88

(54) **SESQUITERPENE SYNTHASES AND METHODS OF USE**

(30) Priority: 04.10.2002 US 415765 P; 02.12.2002 WO PCT/IB02/05070
(62) Divisional of application: 10177152.5
(71) Applicant: Firmenich S.A., 1211 Geneva 8 (CH)
(72) Inventor: SCHALK, Michel, 1211 Geneva 8 (CH); CLARK, Antony, Hopewell, NJ 08525 (US)
(74) Representative: Armstrong, Iain Cheshire

(57) **Abstract**

The invention relates to sesquiterpene synthases and methods of their production and use. In one embodiment, the invention provides nucleic acids comprising a nucleotide sequence as described herein that encodes for at least one sesquiterpene synthases. In a further embodiment, the invention also provides for sesquiterpene synthases and methods of making and using these enzymes. For example, sesquiterpene synthases of the invention may be used to convert farnesyl-pyrophosphate to various oxygenated and aliphatic sesquiterpenes including valencene, bicyclo-germacrene, cubebol and delta- cadinene.

SEQUENCE LISTING

<110>  Firmenich SA
       Michel, Schalk
       Anthony, Clark

<120>  Sesquiterpene Synthases and Methods of Use

<130>  5770-EUR-D1

<140>  10177152.5 (divisional of PCT/IB/03/005072)
<141>  2003-10-02

<150>  US 60/415,765
<151>  2002-10-04

<150>  PCT/IB02/05070
<151>  2002-12-02

<160>  65

<170>  PatentIn version 3.1

<210>  1
<211>  556
<212>  PRT
<213>  Citrus paradisi

<400>  1

Met Ala Leu Gln Asp Ser Glu Val Pro Ser Ser Ile Leu Asn Ala Thr
1               5                   10                  15

Ala Gly Asn Arg Pro Thr Ala Ser Tyr His Pro Thr Leu Trp Gly Gly
            20                  25                  30

Lys Phe Leu Asp Tyr Ser Ser Val Asp Asp Ser Glu Ala Met Asp Ala
        35                  40                  45

Thr Ile Asp Gln Asp Glu Phe Glu Ala Leu Lys Gln Lys Ile Lys Asn
    50                  55                  60

Met Leu Ile Ser Pro Thr Asp Lys Ser Phe Gln Lys Leu Asn Leu Ile
65                  70                  75                  80

Asp Ala Val Gln Arg Leu Gly Val Ala Tyr His Phe Glu Arg Glu Ile
                85                  90                  95

Glu Asp Glu Leu Glu Lys Leu Ser Pro Asp Glu Tyr Asp Gly Asn Asp
            100                 105                 110

Val His Ser Val Ala Leu Arg Phe Arg Leu Leu Arg Gln Gln Gly Tyr
        115                 120                 125

Arg Ile Ser Cys Asp Ile Phe Gly Gly Phe Lys Asp Asp Arg Gly Lys

130                 135                 140

Phe Lys Val Ser Leu Ile Asn Asp Val Thr Gly Met Leu Ser Leu Tyr
145                 150                 155                 160

Glu Ala Ala His Leu Arg Ile Arg Gly Glu Asp Ile Leu Asp Glu Ala
                165                 170                 175

Leu Ala Phe Thr Thr Ser His Leu Glu Ser Met Val Thr Gln Val Ser
            180                 185                 190

Pro Gln Leu Ser Asp Glu Ile Leu His Ala Leu Asn Arg Pro Ile Arg
        195                 200                 205

Arg Gly Leu Pro Arg Leu Glu Ala Val Tyr Tyr Ile Asp Leu Tyr Ser
    210                 215                 220

Arg Asp Asp Ser Lys Asp Lys Ala Ile Leu Leu Lys Phe Ala Lys Leu
225                 230                 235                 240

Asp Phe Cys Met Leu Gln Val Ile His Arg Lys Glu Leu Ser Ile Ile
                245                 250                 255

Thr Glu Trp Trp Lys Asn Leu Asp Val Glu Ile Asn Leu Pro Tyr Ala
            260                 265                 270

Arg Asn Arg Val Val Glu Cys Tyr Phe Trp Ala Met Gly Val Tyr Phe
        275                 280                 285

Glu Pro Arg Tyr Ser Phe Ala Arg Lys Ile Leu Ser Lys Val Ile Ala
    290                 295                 300

Met Ala Ser Ile Leu Asp Asp Thr Tyr Asp Ala Tyr Gly Thr Leu Glu
305                 310                 315                 320

Glu Leu Glu Leu Phe Thr Asn Ala Ile Lys Arg Trp Asp Ile Ser Asn
                325                 330                 335

Ile Asp Val Leu Pro Lys Tyr Met Lys Leu Ile Tyr Gln Gly Leu Leu
            340                 345                 350

Asp Val Phe Gly Glu Ala Glu Glu Glu Ile Ser Lys Glu Gly Gln Thr
        355                 360                 365

Tyr Cys Met Ser Tyr Val Ile Gln Ala Val Lys Lys Val Val Gln Ala
    370                 375                 380

Tyr Phe Glu Glu Ala Lys Trp Cys Ser Glu Gly Tyr Phe Pro Lys Val
385                 390                 395                 400

Glu Glu Tyr Met Gln Val Ser Leu Val Thr Thr Cys Tyr His Met Leu
                405                 410                 415

Ala Thr Ala Ser Phe Leu Gly Met Gly Lys Ile Ala Asp Lys Gln Ala
            420                 425                 430

Phe Glu Trp Ile Ser Asn Tyr Pro Lys Thr Val Lys Ala Ser Gln Val
        435                 440                 445

Ile Cys Arg Leu Met Asp Asp Ile Val Ser His Glu Phe Glu Gln Lys
    450                 455                 460

Arg Lys His Val Ala Ser Gly Ile Glu Cys Tyr Met Lys Gln His Gly
465                 470                 475                 480

Val Ser Asp Glu Glu Val Ile Lys Val Phe Arg Lys Gln Ile Ser Asn
                485                 490                 495

Gly Trp Lys Asp Val Asn Glu Gly Phe Met Lys Pro Thr Glu Val Ala
            500                 505                 510

Met Pro Leu Leu Glu Arg Ile Leu Asn Leu Ala Arg Val Ile Asp Val
        515                 520                 525

Ile Tyr Lys Asp Asp Asp Gly Tyr Thr Asn Ser Tyr Val Ile Lys Asp
    530                 535                 540

Tyr Ile Ala Thr Leu Leu Glu Lys Pro Val Pro Phe
545                 550                 555

<210>  2
<211>  562
<212>  PRT
<213>  Citrus paradisi

<400>  2

Met Ser Leu Glu Val Ser Ala Ser Pro Ala Lys Val Ile Gln Asn Ala
1               5                   10                  15

Gly Lys Asp Ser Thr Arg Arg Ser Ala Asn Tyr His Pro Ser Ile Trp
            20                  25                  30

Gly Asp His Phe Leu Gln Tyr Thr Cys Asp Thr Gln Glu Thr Asp Asp
        35                  40                  45

Gly Ser Asn Val Lys His Leu Glu Leu Lys Lys Glu Ile Arg Arg Met
    50                  55                  60

Leu Lys Ala Asp Asn Lys Pro Ser Arg Thr Leu Gln Leu Ile Asp Ala
65                  70                  75                  80

Ile Gln Arg Leu Gly Val Ser Tyr His Phe Glu Ser Glu Ile Asp Glu
                85                  90                  95

Ile Leu Gly Lys Met His Lys Ala Ser Gln Asp Ser Asp Leu Cys Asp
            100                 105                 110

Asn Glu Asn Asp Glu Leu Tyr Tyr Ile Ser Leu His Phe Arg Leu Leu
    115                 120                 125

Arg Gln Asn Gly Tyr Lys Ile Ser Ala Asp Val Phe Lys Lys Phe Lys
    130                 135                 140

Asp Thr Asp Gly Asn Phe Lys Thr Ser Leu Ala Lys Asp Val Arg Gly
145                 150                 155                 160

Met Leu Ser Leu Tyr Glu Ala Thr His Leu Gly Val His Glu Glu Asp
                165                 170                 175

Ile Leu Asp Glu Ala Leu Ala Phe Thr Thr Ser His Leu Glu Ser Ile
            180                 185                 190

Ala Thr His Gln Ile Arg Ser Pro Leu Val Glu Gln Val Lys His Ala
        195                 200                 205

Leu Val Gln Pro Ile His Arg Gly Phe Gln Arg Leu Glu Ala Arg Gln
    210                 215                 220

Tyr Ile Pro Ile Tyr Gln Glu Glu Ser Pro His Asn Glu Ala Leu Leu
225                 230                 235                 240

Thr Phe Ala Lys Leu Asp Phe Asn Lys Leu Gln Lys Pro His Gln Lys
                245                 250                 255

Glu Leu Gly Asp Ile Ser Arg Trp Trp Lys Glu Leu Asp Phe Ala His
            260                 265                 270

Lys Leu Pro Phe Ile Arg Asp Arg Val Ala Glu Cys Tyr Phe Trp Ile
        275                 280                 285

Leu Gly Val Tyr Phe Glu Pro Gln Tyr Ser Phe Ala Arg Arg Ile Leu
    290                 295                 300

Thr Lys Val Ile Ser Met Thr Ser Val Ile Asp Asp Ile Tyr Asp Val
305                 310                 315                 320

Tyr Gly Lys Ile Glu Glu Leu Glu Leu Phe Thr Ser Ala Ile Glu Arg
                325                 330                 335

Trp Asp Ile Ser Ala Ile Asp Gln Leu Pro Glu Tyr Met Lys Leu Cys
            340                 345                 350

Tyr Arg Ala Leu Leu Asp Val Phe Ser Glu Ala Glu Lys Asp Leu Ala
        355                 360                 365

Pro Gln Gly Lys Ser Tyr Arg Leu Tyr Tyr Ala Lys Glu Ala Met Lys
    370                 375                 380

Asn Met Val Lys Asn Tyr Phe Tyr Glu Ala Lys Trp Cys Leu Gln Asn
385                 390                 395                 400

Tyr Val Pro Thr Val Asp Glu Tyr Met Thr Val Ala Leu Val Thr Ser
                405                 410                 415

Gly Ser Pro Met Leu Ser Thr Thr Ser Phe Val Gly Met Gly Asp Ile
            420                 425                 430

Val Thr Lys Glu Ser Phe Glu Trp Leu Phe Ser Asn Pro Arg Phe Ile
        435                 440                 445

Arg Ala Ser Ser Ile Val Cys Arg Leu Met Asp Asp Ile Val Ser His
    450                 455                 460

Lys Phe Glu Gln Ser Arg Gly His Val Ala Ser Ser Val Glu Cys Tyr
465                 470                 475                 480

Met Lys Gln His Gly Ala Thr Glu Glu Glu Ala Cys Asn Glu Phe Arg
                485                 490                 495

Lys Gln Val Ser Asn Ala Trp Lys Asp Ile Asn Glu Asp Cys Leu Arg
            500                 505                 510

Pro Thr Val Val Pro Met Pro Leu Leu Met Arg Ile Leu Asn Leu Thr
        515                 520                 525

Arg Val Ile Asp Val Ile Tyr Lys Tyr Glu Asp Gly Tyr Thr His Ser
    530                 535                 540

Ala Val Val Leu Lys Asp Phe Val Ala Ser Leu Phe Ile Asn Pro Val
545                 550                 555                 560

Pro Ile

<210> 3
<211> 555
<212> PRT
<213> Citrus paradisi

<400> 3

Met Ser Ala Gln Val Leu Ala Thr Val Ser Ser Ser Thr Glu Lys Thr
1               5                   10                  15

Val Arg Pro Ile Ala Gly Phe His Pro Asn Leu Trp Gly Asp Tyr Phe
            20                  25                  30

Leu Thr Leu Ala Ser Asp Cys Lys Thr Asp Asp Thr Thr His Gln Glu
        35                  40                  45

Glu Tyr Glu Ala Leu Lys Gln Glu Val Arg Ser Met Ile Thr Ala Thr
    50                  55                  60

Ala Asp Thr Pro Ala Gln Lys Leu Gln Leu Val Asp Ala Val Gln Arg
65                  70                  75                  80

Leu Gly Val Ala Tyr His Phe Glu Gln Glu Ile Glu Asp Ala Met Glu
                85                  90                  95

Lys Ile Tyr His Asp Asp Phe Asp Asn Asn Asp Asp Val Asp Leu Tyr
            100                 105                 110

Thr Val Ser Leu Arg Phe Arg Leu Leu Arg Gln Gln Gly Phe Lys Val
        115                 120                 125

Pro Cys Asp Val Phe Ala Lys Phe Lys Asp Asp Glu Gly Lys Phe Lys
    130                 135                 140

Ala Ser Leu Val Arg Asp Val His Gly Ile Leu Ser Leu Tyr Glu Ala
145                 150                 155                 160

Gly His Leu Ala Ile Arg Gly Glu Gly Ile Leu Asp Glu Ala Ile Ala
                165                 170                 175

Phe Thr Arg Thr His Leu Gln Ser Met Val Ser Gln Asp Val Cys Pro
            180                 185                 190

Asn Asn Leu Ala Glu Gln Ile Asn His Thr Leu Asp Cys Pro Leu Arg
        195                 200                 205

Arg Ala Leu Pro Arg Val Glu Thr Arg Phe Phe Leu Ser Val Tyr Pro
    210                 215                 220

Arg Asp Asp Lys His Asp Lys Thr Leu Leu Lys Phe Ser Lys Leu Asp
225                 230                 235                 240

Phe Asn Leu Val Gln Arg Ile His Gln Lys Glu Leu Ser Ala Ile Thr
                245                 250                 255

Arg Trp Trp Lys Asp Leu Asp Phe Thr Thr Lys Leu Pro Tyr Ala Arg
            260                 265                 270

Asp Arg Ile Val Glu Leu Tyr Phe Trp Ile Val Gly Thr Tyr Phe Glu
        275                 280                 285

Pro Lys Tyr Thr Leu Ala Arg Lys Ile Met Thr Lys Thr Ile Tyr Thr
    290                 295                 300

Ala Ser Ile Ile Asp Asp Thr Phe Asp Ala Tyr Gly Phe Phe Glu Glu
305                 310                 315                 320

Leu Lys Leu Phe Ala Glu Ala Val Gln Arg Trp Asp Ile Gly Ala Met
                325                 330                 335

Asp Ile Leu Pro Glu Tyr Met Lys Val Leu Tyr Lys Ala Leu Leu Asp
            340                 345                 350

Thr Phe Asn Glu Ile Glu Gln Asp Leu Ala Lys Glu Gly Arg Ser Ser
        355                 360                 365

Tyr Leu Pro Tyr Gly Lys Glu Lys Met Gln Glu Leu Val Gln Met Tyr
    370                 375                 380

Phe Val Gln Ala Lys Trp Phe Ser Glu Gly Tyr Val Pro Thr Trp Asp
385                 390                 395                 400

Glu Tyr Tyr Pro Val Gly Leu Val Ser Cys Gly Tyr Phe Met Leu Ala
                405                 410                 415

Thr Asn Ser Phe Leu Gly Met Cys Asp Val Ala Asn Glu Glu Ala Phe
            420                 425                 430

Glu Trp Ile Ser Lys Asp Pro Lys Ile Ser Thr Ala Ser Ser Val Ile
        435                 440                 445

Cys Arg Leu Arg Asn Asp Ile Val Ser His Gln Phe Glu Gln Lys Arg

450                 455                 460

Gly His Ile Ala Ser Gly Phe Glu Cys Tyr Ile Lys Gln Tyr Gly Val
465                 470                 475                 480

Ser Glu Glu Glu Val Val Thr Val Phe Thr Glu Glu Val Glu Asn Ala
                485                 490                 495

Trp Lys Asp Met Asn Glu Glu Phe Leu Lys Pro Thr Ala Phe Pro Val
            500                 505                 510

Ala Leu Ile Glu Arg Pro Phe Asn Ile Ala Arg Val Ile Glu Phe Leu
        515                 520                 525

Asn Lys Lys Gly Asp Trp Tyr Thr His Ser His Ala Ile Lys Asp Gln
    530                 535                 540

Ile Ala Ala Val Leu Arg Asp Pro Val Thr Ile
545                 550                 555

<210>  4
<211>  548
<212>  PRT
<213>  Citrus paradisi

<400>  4

Met Ser Ser Gly Glu Thr Phe Arg Pro Thr Ala Asp Phe His Pro Ser
1               5                   10                  15

Leu Trp Arg Asn His Phe Leu Lys Gly Ala Ser Asp Phe Lys Thr Val
            20                  25                  30

Asp His Thr Ala Thr Gln Glu Arg His Glu Ala Leu Lys Glu Glu Val
        35                  40                  45

Arg Arg Met Ile Thr Asp Ala Glu Asp Lys Pro Val Gln Lys Leu Arg
    50                  55                  60

Leu Ile Asp Glu Val Gln Arg Leu Gly Val Ala Tyr His Phe Glu Lys
65                  70                  75                  80

Glu Ile Glu Asp Ala Ile Gln Lys Leu Cys Pro Ile Tyr Ile Asp Ser
                85                  90                  95

Asn Arg Ala Asp Leu His Thr Val Ser Leu His Phe Arg Leu Leu Arg
            100                 105                 110

Gln Gln Gly Ile Lys Ile Ser Cys Asp Val Phe Glu Lys Phe Lys Asp

        115                 120                 125

Asp Glu Gly Arg Phe Lys Ser Ser Leu Ile Asn Asp Val Gln Gly Met
    130                 135                 140

Leu Ser Leu Tyr Glu Ala Ala Tyr Met Ala Val Arg Gly Glu His Ile
145                 150                 155                 160

Leu Asp Glu Ala Ile Ala Phe Thr Thr Thr His Leu Lys Ser Leu Val
                165                 170                 175

Ala Gln Asp His Val Thr Pro Lys Leu Ala Glu Gln Ile Asn His Ala
            180                 185                 190

Leu Tyr Arg Pro Leu Arg Lys Thr Leu Pro Arg Leu Glu Ala Arg Tyr
        195                 200                 205

Phe Met Ser Met Ile Asn Ser Thr Ser Asp His Leu Tyr Asn Lys Thr
    210                 215                 220

Leu Leu Asn Phe Ala Lys Leu Asp Phe Asn Ile Leu Leu Glu Leu His
225                 230                 235                 240

Lys Glu Glu Leu Asn Glu Leu Thr Lys Trp Trp Lys Asp Leu Asp Phe
                245                 250                 255

Thr Thr Lys Leu Pro Tyr Ala Arg Asp Arg Leu Val Glu Leu Tyr Phe
            260                 265                 270

Trp Asp Leu Gly Thr Tyr Phe Glu Pro Gln Tyr Ala Phe Gly Arg Lys
        275                 280                 285

Ile Met Thr Gln Leu Asn Tyr Ile Leu Ser Ile Ile Asp Asp Thr Tyr
    290                 295                 300

Asp Ala Tyr Gly Thr Leu Glu Glu Leu Ser Leu Phe Thr Glu Ala Val
305                 310                 315                 320

Gln Arg Trp Asn Ile Glu Ala Val Asp Met Leu Pro Glu Tyr Met Lys
                325                 330                 335

Leu Ile Tyr Arg Thr Leu Leu Asp Ala Phe Asn Glu Ile Glu Glu Asp
            340                 345                 350

Met Ala Lys Gln Gly Arg Ser His Cys Val Arg Tyr Ala Lys Glu Glu
        355                 360                 365

Asn Gln Lys Val Ile Gly Ala Tyr Ser Val Gln Ala Lys Trp Phe Ser
    370                 375                 380

Glu Gly Tyr Val Pro Thr Ile Glu Glu Tyr Met Pro Ile Ala Leu Thr
385                 390                 395                 400

Ser Cys Ala Tyr Thr Phe Val Ile Thr Asn Ser Phe Leu Gly Met Gly
                405                 410                 415

Asp Phe Ala Thr Lys Glu Val Phe Glu Trp Ile Ser Asn Asn Pro Lys
            420                 425                 430

Val Val Lys Ala Ala Ser Val Ile Cys Arg Leu Met Asp Asp Met Gln
        435                 440                 445

Gly His Glu Phe Glu Gln Lys Arg Gly His Val Ala Ser Ala Ile Glu
    450                 455                 460

Cys Tyr Thr Lys Gln His Gly Val Ser Lys Glu Glu Ala Ile Lys Met
465                 470                 475                 480

Phe Glu Glu Glu Val Ala Asn Ala Trp Lys Asp Ile Asn Glu Glu Leu
                485                 490                 495

Met Met Lys Pro Thr Val Val Ala Arg Pro Leu Leu Gly Thr Ile Leu
            500                 505                 510

Asn Leu Ala Arg Ala Ile Asp Phe Ile Tyr Lys Glu Asp Asp Gly Tyr
        515                 520                 525

Thr His Ser Tyr Leu Ile Lys Asp Gln Ile Ala Ser Val Leu Gly Asp
    530                 535                 540

His Val Pro Phe
545

<210>  5
<211>  548
<212>  PRT
<213>  Citrus paradisi

<400>  5

Met Ser Ser Gly Glu Thr Phe Arg Pro Thr Ala Asp Phe His Pro Ser
1               5                   10                  15

Leu Trp Arg Asn His Phe Leu Lys Gly Ala Ser Asp Phe Lys Thr Val
            20                  25                  30

Asp His Thr Ala Thr Gln Glu Arg His Glu Ala Leu Lys Glu Glu Val
        35                  40                  45

Arg Arg Met Ile Thr Asp Ala Glu Asp Lys Pro Val Gln Lys Leu Arg
    50                  55                  60

Leu Ile Asp Glu Val Gln Arg Leu Gly Val Ala Tyr His Phe Glu Lys
65                  70                  75                  80

Glu Ile Glu Asp Ala Ile Gln Lys Leu Cys Pro Asn Tyr Ile His Ser
                85                  90                  95

Asn Ser Pro Asp Leu His Thr Val Ser Leu His Phe Arg Leu Leu Arg
            100                 105                 110

Gln Gln Gly Ile Lys Ile Ser Cys Asp Val Phe Glu Lys Phe Lys Asp
        115                 120                 125

Asp Glu Gly Arg Phe Lys Ser Ser Leu Ile Asn Asp Val Gln Gly Met
    130                 135                 140

Leu Ser Leu Tyr Glu Ala Ala Tyr Met Ala Val Arg Gly Glu His Ile
145                 150                 155                 160

Leu Asp Glu Ala Ile Ala Phe Thr Thr Thr His Leu Lys Ser Leu Val
                165                 170                 175

Ala Gln Asp His Val Thr Pro Lys Leu Ala Glu Gln Ile Asn His Ala
            180                 185                 190

Leu Tyr Arg Pro Leu Arg Lys Thr Leu Pro Arg Leu Glu Ala Arg Tyr
        195                 200                 205

Phe Met Ser Met Ile Asn Ser Thr Ser Asp His Leu Tyr Asn Lys Thr
    210                 215                 220

Leu Leu Asn Phe Ala Lys Leu Asp Phe Asn Ile Leu Leu Glu Leu His
225                 230                 235                 240

Lys Glu Glu Leu Asn Glu Leu Thr Lys Trp Trp Lys Asp Leu Asp Phe
                245                 250                 255

Thr Thr Lys Leu Pro Tyr Ala Arg Asp Arg Leu Val Glu Leu Tyr Phe
            260                 265                 270

Trp Asp Leu Gly Thr Tyr Phe Glu Pro Gln Tyr Ala Phe Gly Arg Lys
        275                 280                 285

Ile Met Thr Gln Leu Asn Tyr Ile Leu Ser Ile Ile Asp Asp Thr Tyr
    290                 295                 300

Asp Ala Tyr Gly Thr Leu Glu Glu Leu Ser Leu Phe Thr Glu Ala Val
305                 310                 315                 320

Gln Arg Trp Asn Ile Glu Ala Val Asp Met Leu Pro Glu Tyr Met Lys
                325                 330                 335

Leu Ile Tyr Arg Thr Leu Leu Asp Ala Phe Asn Glu Ile Glu Glu Asp
            340                 345                 350

Met Ala Lys Gln Gly Arg Ser His Cys Val Arg Tyr Ala Lys Glu Glu
        355                 360                 365

Asn Gln Lys Val Ile Gly Ala Tyr Ser Val Gln Ala Lys Trp Phe Ser
    370                 375                 380

Glu Gly Tyr Val Pro Thr Ile Glu Glu Tyr Met Pro Ile Ala Leu Thr
385                 390                 395                 400

Ser Cys Ala Tyr Thr Phe Val Ile Thr Asn Ser Phe Leu Gly Met Gly
                405                 410                 415

Asp Phe Ala Thr Lys Glu Val Phe Glu Trp Ile Ser Asn Asn Pro Lys
            420                 425                 430

Val Val Lys Ala Ala Ser Val Ile Cys Arg Leu Met Asp Asp Met Gln
        435                 440                 445

Gly His Glu Phe Glu Gln Lys Arg Gly His Val Ala Ser Ala Ile Glu
    450                 455                 460

Cys Tyr Thr Lys Gln His Gly Val Ser Lys Glu Glu Ala Ile Lys Met
465                 470                 475                 480

Phe Glu Glu Glu Val Ala Asn Ala Trp Lys Asp Ile Asn Glu Glu Leu
                485                 490                 495

Met Met Lys Pro Thr Val Val Ala Arg Pro Leu Leu Gly Thr Ile Leu
            500                 505                 510

Asn Leu Ala Arg Ala Ile Asp Phe Ile Tyr Lys Glu Asp Asp Gly Tyr
        515                 520                 525

Thr His Ser Tyr Leu Ile Lys Asp Gln Ile Ala Ser Val Leu Gly Asp
    530                 535                 540

His Val Pro Phe
545

<210> 6
<211> 1671
<212> DNA
<213> Citrus paradisi

<400> 6
atggcacttc aagattcaga agttccttct tccattctga atgctacagc cggcaaccgt 60

cccacagcta gttatcatcc caccctctgg gggggaaaat tccttgacta ttcttctgtt 120

gacgactctg aggcaatgga tgccacaatt gatcaagacg aatttgaagc acttaagcaa 180

aaaataaaga acatgttaat ctcaccaacc gataagtctt ttcaaaaatt gaacttgatt 240

gatgccgtcc aacgcttagg agtggcttac cattttgaga gggagataga agatgaacta 300

gaaaaactat ctcctgatga gtatgatggc aacgatgtac actccgttgc tcttcgattt 360

cggttactca gacaacaagg atatcgcata tcatgcgata tttttggcgg tttcaaagat 420

gatcgaggaa agttcaaggt atccttaatt aatgatgtga ccggcatgct aagtttgtat 480

gaggctgcac atcttcgcat tcgcggggaa gatatcctgg atgaagccct agctttcact 540

acttctcacc tggaatcaat ggttactcaa gtaagccctc agctttctga tgaaatactt 600

catgccttga ataggccaat ccgcagaggc ttaccaaggc tggaggcagt ctattacatc 660

gatctctact cacgagatga ttcaaaggat aaagcaatat tactaaagtt tgcaaaacta 720

gatttttgca tgcttcaagt aattcaccgt aaggagttaa gtatcatcac agagtggtgg 780

aaaaatttag atgttgaaat aaatctccca tatgctagaa acagagttgt agaatgctat 840

ttttgggcaa tgggagtgta ttttgagcct cgatactcct ttgcaagaaa gatattgtcc 900

aaagtaattg caatggcatc cattttagat gatacctacg acgcctatgg cacacttgaa 960

gaacttgagc tctttacaaa tgctatcaaa aggtgggata ttagcaacat agatgtactt 1020

ccgaagtaca tgaaactgat ttatcaagga ctcttggatg tttttggtga agctgaggag 1080

gaaatctcaa aggaaggaca gacatattgc atgtcatatg tcatacaagc ggtgaagaaa 1140

gtagtccaag cctactttga ggaagccaag tggtgcagtg aaggttattt tccaaaagtg 1200

gaggagtata tgcaagtttc acttgtgaca acttgctatc atatgctggc aacggcttct 1260

tttcttggca tgggaaagat tgctgataag caggcctttg aatggatctc caattaccct 1320

aaaactgtga aagcctccca agttatttgc agacttatgg atgatatagt gtctcacgag 1380

tttgaacaaa aaagaaagca tgttgcctcg ggtattgaat gttacatgaa gcagcatggc 1440

gtctctgatg aagaggtaat taaagtattc cgcaaacaaa tatcaaatgg atggaaagat 1500

gtaaatgaag gattcatgaa gccaacagaa gtggcaatgc ctctccttga gcgcattctc 1560

aatcttgcac gagtgataga tgttatttac aaggatgatg atggctacac caactcttat 1620

gtgatcaaag actacatcgc cacattgctt gagaagcctg ttcccttttg a 1671

<210> 7
<211> 1689
<212> DNA
<213> Citrus paradisi

<400> 7
atgtctttgg aagtttcagc ctctcctgct aaagttatcc aaaatgctgg gaaagattct 60

actcgtcgct ctgcaaatta tcatccaagc atctgggggg atcatttcct tcaatatact 120

tgtgacaccc aggaaactga tgatggcagc aatgtaaagc atctagagct gaagaaagaa 180

attagaagaa tgctaaaagc tgataacaag ccttcacgta cacttcaatt gattgatgca 240

attcagcgtt taggagtgtc ttaccatttt gaaagtgaga ttgatgaaat attgggaaag 300

atgcataagg cttcccaaga ctctgatctt tgtgataatg aaaatgatga gctctattat 360

atctctcttc attttcgatt acttagacaa aatggctata aaatttccgc tgatgtgttc 420

aaaaagttca aagacacgga tgggaacttt aaaacatctc ttgcgaaaga tgttcgagga 480

atgttaagct tgtatgaagc tacgcatctc ggggtacatg aagaagatat actagatgaa 540

gcgcttgctt tcaccactag tcacctagag tcaatagcga ctcatcaaat caggtctcca 600

cttgttgaac aagtcaaaca tgccttagtt cagcctatcc acaggggctt ccaaaggctt 660

gaggcaagac agtacattcc tatctatcaa gaagaatctc cccacaatga agctctgtta 720

acttttgcaa agttagattt taacaaattg caaaagcctc accagaagga actcggtgat 780

atttcaaggt ggtggaaaga attagacttt gcacataagc tacctttcat aagagataga 840

gttgcggagt gctacttttg gatattagga gtgtatttcg agccccaata ttcatttgca 900

agaagaatat tgacgaaagt gatctccatg acttctgtta ttgatgatat ctatgatgtg 960

tatggcaaaa ttgaagaact tgagcttttt acttcagcta ttgagaggtg ggatatcagt 1020

gccatagatc aacttcctga gtatatgaaa ttgtgttata gggcccttct tgatgttttt 1080

agtgaagcag agaaggattt ggccccccaa ggaaaatcat accgcctcta ttatgcaaaa 1140

gaagcgatga agaatatggt taagaattac ttctacgaag ctaaatggtg tcttcagaat 1200

tatgtaccta cagtggatga gtacatgacg gttgcattag ttacatctgg ctccccaatg 1260

ttgtcaacca catcctttgt tggcatggga gacattgtaa ctaaagaatc ttttgagtgg 1320

ttattcagca atcctagatt tattagggct tcttctatag tttgccgact catggatgac 1380

atagtgtcac acaagtttga acaaagcaga gggcacgttg cctcaagcgt tgagtgttac 1440

atgaaacaac atggagcaac agaagaggaa gcatgcaatg agtttcggaa acaagtttca 1500

aatgcctgga aggatataaa tgaggactgc ctacgcccaa cggttgtgcc aatgccactt 1560

ctgatgcgaa ttctcaatct tacacgcgtt atagatgtca tttacaagta tgaagatggc 1620

tacactcatt ccgcagttgt gctgaaagat tttgttgctt ctttgtttat taatcctgtg 1680

ccgatatga 1689

<210> 8
<211> 1668
<212> DNA
<213> Citrus paradisi

<400> 8
atgtccgctc aagttctagc aacggtttcc agttcgacag aaaaaactgt tcgtcccatt 60

gctggtttcc atcctaactt atggggagac tatttcctga ccctcgcttc tgattgcaag 120

acagatgata ctacgcacca agaggaatac gaagcgctga agcaagaagt cagaagcatg 180

ataacggcta cggcagatac acctgcccag aagttgcaat tggttgatgc agtccaacga 240

ttgggtgtgg cctatcactt cgaacaggag atagaagatg caatggaaaa gatttatcac 300

gatgactttg ataataacga tgatgtcgat ctctacactg tttctcttcg ttttcgactg 360

cttaggcagc aaggatttaa ggttccgtgt gatgtgttcg cgaagttcaa agatgatgaa 420

ggtaaattca aggcatcatt ggtgcgggat gttcatggca ttctaagttt gtatgaggca 480

ggacacttgg ccattcgcgg agaagggata ttagatgaag ccattgcttt cactagaact 540

caccttcagt caatggtatc tcaggatgta tgccctaata atcttgctga acaaattaat 600

catactctcg actgtcctct ccgcagagcc cttccaagag tggagacaag atttttcttg 660

tcggtctatc caagagatga taaacacgat aaaactttgt taaagttttc aaagttagac 720

tttaaccttg tgcaaagaat acatcagaag gaattaagtg ccatcacacg gtggtggaaa 780

gatttagact tcactacaaa gctaccttat gcaagagaca gaatcgtaga gttgtatttt 840

tggattgtag ggacgtattt tgaaccaaag tacactttag caagaaaaat aatgaccaaa 900

acaatttaca cggcatctat catagatgac actttcgacg cttatggttt ctttgaagag 960

ctcaaactct ttgcagaagc agtccagagg tgggacattg gagccatgga tatacttcca 1020

gaatacatga aagtgcttta taaggccctt ttagatactt tcaatgaaat tgagcaagac 1080

ttggccaagg aaggaagatc gtcctactta ccttatggca aagaaaagat gcaagagctt 1140

gttcaaatgt actttgttca agccaagtgg ttcagtgaag gttatgttcc gacatgggac 1200

gaatattatc cggttggact tgtaagttgc ggctacttca tgcttgcgac aaactccttc 1260

cttggcatgt gtgatgttgc aaacgaggaa gcttttgaat ggatatccaa ggaccctaag 1320

atttcaacag cgtcatcagt tatctgcaga cttaggaatg acattgtttc ccaccagttt 1380

gaacagaaga gaggacatat tgcctcagga tttgaatgct acattaagca gtatggtgtt 1440

tcagaagaag aggtagttac agtttttact gaagaagttg agaatgcatg gaaagatatg 1500

aatgaggaat tcctgaaacc aactgctttt cctgtggctt tgattgagag acctttcaat 1560

atcgcacgtg tgattgaatt tctaaacaag aagggtgatt ggtacactca ttctcatgcg 1620

attaaagacc agattgccgc agtgctcaga gaccctgtta ccatctga 1668

<210> 9
<211> 1647
<212> DNA
<213> Citrus paradisi

<400> 9
atgtcgtctg gagaaacatt tcgtcctact gcagatttcc atcctagttt atggagaaac 60

catttcctca aaggtgcttc tgatttcaag acagttgatc atactgcaac tcaagaacga 120

cacgaggcac tgaaagaaga ggtaaggaga atgataacag atgctgaaga taagcctgtt 180

cagaagttac gcttgattga tgaagtacaa cgcctggggg tggcttatca ctttgagaaa 240

gaaatagaag atgcaataca aaaattatgt ccaatctata ttgacagtaa tagagctgat 300

ctccacaccg tttcccttca ttttcgattg cttaggcagc aaggaatcaa gatttcatgt 360

gatgtgtttg agaagttcaa agatgatgag ggtagattca agtcatcgtt gataaacgat 420

gttcaaggga tgttaagttt gtacgaggca gcatacatgg cagttcgcgg agaacatata 480

ttagatgaag ccattgcttt cactaccact cacctgaagt cattggtagc tcaggatcat 540

gtaaccccta agcttgcgga acagataaat catgctttat accgtcctct tcgtaaaacc 600

ctaccaagat tagaggcgag gtattttatg tccatgatca attcaacaag tgatcattta 660

tacaataaaa ctctgctgaa ttttgcaaag ttagatttta acatattgct agagctgcac 720

aaggaggaac tcaatgaatt aacaaagtgg tggaaagatt tagacttcac tacaaaacta 780

ccttatgcaa gagacagatt agtggagtta tatttttggg atttagggac atacttcgag 840

cctcaatatg catttgggag aaagataatg acccaattaa attacatatt atccatcata 900

gatgatactt atgatgcgta tggtacactt gaagaactca gcctctttac tgaagcagtt 960

caaagatgga atattgaggc cgtagatatg cttccagaat acatgaaatt gatttacagg 1020

acactcttag atgcttttaa tgaaattgag gaagatatgg ccaagcaagg aagatcacac 1080

tgcgtacgtt atgcaaaaga ggagaatcaa aaagtaattg gagcatactc tgttcaagcc 1140

aaatggttca gtgaaggtta cgttccaaca attgaggagt atatgcctat tgcactaaca 1200

agttgtgctt acacattcgt cataacaaat tccttccttg gcatgggtga ttttgcaact 1260

aaagaggttt ttgaatggat ctccaataac cctaaggttg taaaagcagc atcagttatc 1320

tgcagactca tggatgacat gcaaggtcat gagtttgagc agaagagagg acatgttgcg 1380

tcagctattg aatgttacac gaagcagcat ggtgtctcta aggaagaggc aattaaaatg 1440

tttgaagaag aagttgcaaa tgcatggaaa gatattaacg aggagttgat gatgaagcca 1500

accgtcgttg cccgaccact gctcgggacg attcttaatc ttgctcgtgc aattgatttt 1560

atttacaaag aggacgacgg ctatacgcat tcttacctaa ttaaagatca aattgcttct 1620

gtgctaggag accacgttcc attttga 1647

<210> 10
<211> 1647
<212> DNA
<213> Citrus paradisi

<400> 10
atgtcgtctg gagaaacatt tcgtcctact gcagatttcc atcctagttt atggagaaac 60

catttcctca aaggtgcttc tgatttcaag acagttgatc atactgcaac tcaagaacga 120

cacgaggcac tgaaagaaga ggtaaggaga atgataacag atgctgaaga taagcctgtt 180

cagaagttac gcttgattga tgaagtacaa cgcctggggg tggcttatca ctttgagaaa 240

gaaatagaag atgcaataca aaaattatgt ccaaactata ttcacagtaa tagccctgat 300

cttcacaccg tttctcttca ttttcgattg cttaggcagc aaggaatcaa gatttcatgt 360

gatgtgtttg agaagttcaa agatgatgag ggtagattca agtcatcgtt gataaacgat 420

gttcaaggga tgttaagttt gtacgaggca gcatacatgg cagttcgcgg agaacatata 480

ttagatgaag ccattgcttt cactaccact cacctgaagt cattggtagc tcaggatcat 540

gtaaccccta agcttgcgga acagataaat catgctttat accgtcctct tcgtaaaacc 600

ctaccaagat tagaggcgag gtattttatg tccatgatca attcaacaag tgatcattta 660

tacaataaaa ctctgctgaa ttttgcaaag ttagatttta acatattgct agagctgcac 720

aaggaggaac tcaatgaatt aacaaagtgg tggaaagatt tagacttcac tacaaaacta 780

ccttatgcaa gagacagatt agtggagtta tatttttggg atttagggac atacttcgag 840

cctcaatatg catttgggag aaagataatg acccaattaa attacatatt atccatcata 900

gatgatactt atgatgcgta tggtacactt gaagaactca gcctctttac tgaagcagtt 960

caaagatgga atattgaggc cgtagatatg cttccagaat acatgaaatt gatttacagg 1020

acactcttag atgcttttaa tgaaattgag gaagatatgg ccaagcaagg aagatcacac 1080

tgcgtacgtt atgcaaaaga ggagaatcaa aaagtaattg gagcatactc tgttcaagcc 1140

aaatggttca gtgaaggtta cgttccaaca attgaggagt atatgcctat tgcactaaca 1200

agttgtgctt acacattcgt cataacaaat tccttccttg gcatgggtga ttttgcaact 1260

aaagaggttt ttgaatggat ctccaataac cctaaggttg taaaagcagc atcagttatc 1320

tgcagactca tggatgacat gcaaggtcat gagtttgagc agaagagagg acatgttgcg 1380

tcagctattg aatgttacac gaagcagcat ggtgtctcta aggaagaggc aattaaaatg 1440

tttgaagaag aagttgcaaa tgcatggaaa gatattaacg aggagttgat gatgaagcca 1500

accgtcgttg cccgaccact gctcgggacg attcttaatc ttgctcgtgc aattgatttt 1560

atttacaaag aggacgacgg ctatacgcat tcttacctaa ttaaagatca aattgcttct 1620

gtgctaggag accacgttcc attttga 1647

<210> 11
<211> 74
<212> PRT
<213> Lycopersicon esculentum

<400> 11

Phe His Gln Arg Glu Leu Ser Asp Leu Thr Arg Trp Trp Lys Asp Leu
1               5                   10                  15

Asp Phe Ala Asn Lys Tyr Pro Tyr Ala Arg Asp Arg Leu Val Glu Cys
            20                  25                  30

Tyr Phe Trp Ile Leu Gly Val Tyr Phe Glu Pro Lys Tyr Ser Arg Ala
        35                  40                  45

Arg Lys Met Met Thr Lys Val Leu Asn Leu Thr Ser Ile Ile Asp Asp
    50                  55                  60

Thr Phe Asp Ala Tyr Ala Thr Phe Asp Glu
65                  70

<210> 12
<211> 74
<212> PRT
<213> Mentha piperita

<400> 12

Leu Tyr Lys Glu Glu Leu Ser Gln Leu Ser Arg Trp Trp Asn Thr Trp
1               5                   10                  15

Asn Leu Lys Ser Lys Leu Pro Tyr Ala Arg Asp Arg Val Val Glu Ala
            20                  25                  30

Tyr Val Trp Gly Val Gly Tyr His Tyr Glu Pro Gln Tyr Ser Tyr Val
        35                  40                  45

Arg Met Gly Leu Ala Lys Gly Val Leu Ile Cys Gly Ile Met Asp Asp
    50                  55                  60

Thr Tyr Asp Asn Tyr Ala Thr Leu Asn Glu
65                  70

<210> 13

<211>  73
<212>  PRT
<213>  Abies grandis

<400>  13

Lys His His Lys Glu Ile Gln Phe Ile Thr Arg Trp Trp Arg Asp Ser
1               5                   10                  15

Gly Ile Ser Gln Leu Asn Phe Tyr Arg Lys Arg His Val Glu Tyr Tyr
            20                  25                  30

Ser Trp Val Val Met Cys Ile Phe Glu Pro Glu Phe Ser Glu Ser Arg
        35                  40                  45

Ile Ala Phe Ala Lys Thr Ala Ile Leu Cys Thr Val Leu Asp Asp Leu
    50                  55                  60

Tyr Asp Thr His Ala Thr Leu His Glu
65                  70


<210>  14
<211>  74
<212>  PRT
<213>  Citrus junos

<400>  14

Ile His Gln Lys Glu Leu Ser Ala Ile Thr Arg Trp Trp Lys Asp Leu
1               5                   10                  15

Asp Phe Thr Thr Lys Leu Pro Tyr Ala Arg Asp Arg Ile Val Glu Leu
            20                  25                  30

Tyr Phe Trp Ile Val Gly Thr Tyr Phe Glu Pro Lys Tyr Thr Leu Ala
        35                  40                  45

Arg Lys Ile Met Thr Lys Thr Ile Tyr Thr Ala Ser Ile Ile Asp Asp
    50                  55                  60

Thr Phe Asp Ala Tyr Gly Phe Phe Glu Glu
65                  70


<210>  15
<211>  73
<212>  PRT
<213>  Nicotiana tabacum

<400>  15

Leu His Lys Gln Glu Leu Ala Gln Val Ser Arg Trp Trp Lys Asp Leu
1               5                   10                  15

Asp Phe Val Thr Thr Leu Pro Tyr Ala Arg Asp Arg Val Val Glu Cys
            20                  25                  30

Tyr Phe Trp Ala Leu Gly Val Tyr Phe Glu Pro Gln Tyr Ser Gln Ala
        35                  40                  45

Arg Val Met Leu Val Lys Thr Ile Ser Met Ile Ser Ile Val Asp Asp
    50                  55                  60

Thr Phe Asp Ala Tyr Gly Thr Val Lys
65                  70

<210> 16
<211> 74
<212> PRT
<213> Capsicum annuum

<400> 16

Leu His Lys Gln Glu Leu Ala Glu Val Ser Arg Trp Trp Lys Asp Leu
1               5                   10                  15

Asn Phe Val Asn Thr Leu Pro Tyr Ala Arg Asp Arg Val Val Glu Cys
            20                  25                  30

Tyr Phe Trp Ala Leu Gly Val Tyr Tyr Glu Pro Gln Tyr Ser Gln Ala
        35                  40                  45

Arg Val Met Leu Val Lys Thr Ile Ala Met Ile Ser Ile Val Asp Asp
    50                  55                  60

Thr Tyr Asp Ala Tyr Gly Thr Val Asp Glu
65                  70

<210> 17
<211> 74
<212> PRT
<213> Solanum tuberosum

<400> 17

Leu His Lys His Glu Leu Ser Glu Val Ser Arg Trp Trp Lys Asp Leu
1               5                   10                  15

Asp Phe Val Thr Thr Leu Pro Tyr Ala Arg Asp Arg Ala Val Glu Cys
            20                  25                  30

Tyr Phe Trp Thr Met Gly Val Tyr Ala Glu Pro Gln Tyr Ser Gln Ala
        35                  40                  45

Arg Val Ile Leu Ala Lys Thr Ile Ala Met Ile Ser Ile Val Asp Asp
    50                  55                  60

Thr Phe Asp Ala Tyr Gly Ile Val Lys Glu
65                  70

<210>  18
<211>  74
<212>  PRT
<213>  Hyoscyamus muticus

<400>  18

Leu His Lys His Glu Leu Ser Glu Val Ser Arg Trp Trp Lys Asp Leu
1               5                   10                  15

Asp Phe Val Thr Thr Leu Pro Tyr Ala Arg Asp Arg Ala Val Glu Cys
            20                  25                  30

Tyr Phe Trp Thr Met Gly Val Tyr Ala Glu Pro Gln Tyr Ser Gln Ala
        35                  40                  45

Arg Val Met Leu Ala Lys Thr Ile Ala Met Ile Ser Ile Val Asp Asp
    50                  55                  60

Thr Phe Asp Ala Tyr Gly Ile Val Lys Glu
65                  70

<210>  19
<211>  86
<212>  PRT
<213>  Gossypium arboreum

<400>  19

Leu Glu Phe Ala Lys Ile Asp Phe Asn Met Val Gln Leu Leu His Arg
1               5                   10                  15

Lys Glu Leu Ser Glu Ile Ser Arg Trp Trp Lys Asp Leu Asp Phe Gln
            20                  25                  30

Arg Lys Leu Pro Tyr Ala Arg Asp Arg Val Val Glu Gly Tyr Phe Trp
        35                  40                  45

Ile Ser Gly Val Tyr Phe Glu Pro Gln Tyr Ser Leu Gly Arg Lys Met
    50                  55                  60

Leu Thr Lys Val Ile Ala Met Ala Ser Ile Val Asp Asp Thr Tyr Asp
65                  70                  75                  80

Ser Tyr Ala Thr Tyr Glu

                85

<210>  20
<211>  86
<212>  PRT
<213>  Artemisia annua

<400>  20

Leu Lys Leu Ala Lys Leu Glu Phe Asn Leu Leu Gln Ser Leu His Lys
1               5                   10                  15

Glu Glu Leu Ser His Val Cys Lys Trp Trp Lys Ala Phe Asp Ile Lys
            20                  25                  30

Lys Asn Ala Pro Cys Leu Arg Asp Arg Ile Val Glu Cys Tyr Phe Trp
        35                  40                  45

Gly Leu Gly Ser Gly Tyr Glu Pro Gln Tyr Ser Arg Ala Arg Val Phe
    50                  55                  60

Phe Thr Lys Ala Val Ala Val Ile Thr Leu Ile Asp Asp Thr Tyr Asp
65                  70                  75                  80

Ala Tyr Gly Thr Tyr Glu
                85

<210>  21
<211>  86
<212>  PRT
<213>  Artemisia annua

<400>  21

Leu Lys Leu Ala Lys Leu Gly Phe Asn Gln Leu Gln Ser Leu His Lys
1               5                   10                  15

Lys Glu Leu Ser Ile Ile Ser Lys Trp Trp Lys Ser Phe Asp Val Ala
            20                  25                  30

Asn Asn Leu Pro Tyr Ala Arg Asn Arg Pro Val Glu Cys Tyr Phe Trp
        35                  40                  45

Ala Leu Ala Val Tyr Phe Glu Pro Gln Tyr Ser Glu Ser Arg Val Phe
    50                  55                  60

Leu Ser Arg Phe Phe Ser Ile Gln Thr Phe Leu Asp Asp Thr Tyr Asp
65                  70                  75                  80

Ala Tyr Gly Thr Tyr Glu
                85

<210>  22
<211>  85
<212>  PRT
<213>  Abies grandis

<400>  22

Leu Glu Leu Ala Lys Leu Asp Phe Asn Ile Leu Gln Cys Thr His Gln
1               5                   10                  15

Lys Glu Leu Gln Ile Ile Ser Arg Trp Phe Ala Asp Ser Ser Ile Ala
            20                  25                  30

Ser Leu Asn Phe Tyr Arg Lys Cys Tyr Val Glu Phe Tyr Phe Trp Met
        35                  40                  45

Ala Ala Ala Ile Ser Glu Pro Glu Phe Ser Gly Ser Arg Val Ala Phe
    50                  55                  60

Thr Lys Ile Ala Ile Leu Met Thr Met Leu Asp Asp Leu Tyr Asp Thr
65                  70                  75                  80

His Gly Thr Leu Asp
                85

<210>  23
<211>  18
<212>  DNA
<213>  Artificial Sequence

<220>
<223>  Primer deduced (Example 1)

<400>  23
hhvthwcmmg gtggtgga                                                    18

<210>  24
<211>  18
<212>  DNA
<213>  Artificial Sequence

<220>
<223>  Primer deduced (Example 1)

<220>
<221>  misc_feature
<222>  (3)..(3)
<223>  can be G, A, C or T

<400>  24
gtngarkbbt atktttgg                                                    18

<210>  25
<211>  22
<212>  DNA
<213>  Artificial Sequence

<220>
<223>  Primer deduced (Example 1)

<400>  25
crtrrktrtc gwadgkgtcr tc                                                22


<210>  26
<211>  25
<212>  DNA
<213>  Artificial Sequence

<220>
<223>  Primer deduced (Example 1)

<400>  26
gckaagwtvg gkttcaathw kbtdc                                             25


<210>  27
<211>  29
<212>  DNA
<213>  Artificial Sequence

<220>
<223>  Primer deduced (Example 1)

<220>
<221>  misc_feature
<222>  (9)..(9)
<223>  can be a, t, g, or c


<400>  27
gggawwgwnw bgttgaakkt tatttttgg                                         29


<210>  28
<211>  23
<212>  DNA
<213>  Artificial Sequence

<220>
<223>  Primer deduced (Example 1)

<220>
<221>  misc_feature
<222>  (8)..(8)
<223>  can be a, t, g, or c


<400>  28
gtwscgtngh gtcgtahgtc atc                                               23


<210>  29
<211>  49
<212>  PRT

<213>  Citrus paradisi

<400>  29

Asp Leu Gly Phe Pro Lys Lys Val Pro Tyr Ala Arg Asp Arg Val Val
1               5                   10                  15

Glu Thr Tyr Ile Trp Met Leu Leu Gly Val Ser Tyr Glu Pro Asn Leu
            20                  25                  30

Ala Phe Gly Arg Ile Phe Ala Ser Lys Val Val Cys Ile Ile Ser Ile
        35                  40                  45

Ile

<210>  30
<211>  27
<212>  PRT
<213>  Citrus paradisi

<400>  30

Ile Val Gly Thr Tyr Phe Glu Pro Lys Tyr Thr Leu Ala Arg Lys Ile
1               5                   10                  15

Met Thr Lys Thr Ile Tyr Thr Ala Ser Ile Ile
            20                  25

<210>  31
<211>  26
<212>  PRT
<213>  Citrus paradisi

<400>  31

Met Gly Val Tyr Phe Glu Pro Arg Tyr Ser Phe Ala Arg Lys Ile Leu
1               5                   10                  15

Ser Lys Val Ile Ala Met Ala Ser Ile Leu
            20                  25

<210>  32
<211>  260
<212>  PRT
<213>  Citrus paradisi

<400>  32

Gln Lys Leu His Met Ile Asp Ala Ala Gln Arg Leu Gly Val Ala Tyr
1               5                   10                  15

His Phe Glu Lys Glu Ile Glu Asp Glu Leu Gly Lys Val Ser His Asp
            20                  25                  30

Leu Asp Ser Asp Asp Leu Tyr Val Val Ser Leu Arg Phe Arg Leu Phe
        35                  40                  45

Arg Gln Gln Gly Val Lys Ile Ser Cys Asp Val Phe Glu Lys Phe Lys
    50                  55                  60

Asp Asp Glu Gly Lys Phe Lys Glu Ser Leu Ile Asn Asp Ile Arg Gly
65                  70                  75                  80

Met Ser Ser Leu Tyr Glu Ala Ala Tyr Leu Ala Ile Arg Gly Glu Asp
                85                  90                  95

Ile Leu Asp Glu Ala Ile Val Phe Thr Thr Thr His Leu Lys Ser Val
            100                 105                 110

Ile Ser Val Ser Asp His Ser His Val Asn Ser Asp Leu Ala Glu Gln
        115                 120                 125

Ile Arg His Ser Leu Gln Ile Pro Leu Arg Lys Ala Ala Ala Arg Leu
    130                 135                 140

Glu Ala Arg Tyr Phe Leu Asp Ile Tyr Ser Arg Asp Asp Leu His Asp
145                 150                 155                 160

Glu Thr Leu Leu Lys Phe Ala Lys Leu Asp Phe Asn Ile Leu Gln Ala
                165                 170                 175

Ala His Lys Lys Glu Ala Ser Ile Met Thr Arg Trp Trp Asn Asp Leu
            180                 185                 190

Gly Phe Pro Lys Lys Val Pro Tyr Ala Arg Asp Arg Val Val Glu Thr
        195                 200                 205

Tyr Ile Trp Met Leu Leu Gly Val Ser Tyr Glu Pro Asn Leu Ala Phe
    210                 215                 220

Gly Arg Ile Phe Ala Ser Lys Val Val Cys Ile Ile Ser Ile Ile Asp
225                 230                 235                 240

Asp Thr Phe Asp Ala Tyr Gly Thr Phe Glu Glu Leu Thr Leu Phe Thr
                245                 250                 255

Glu Ala Val Thr
            260

<210>  33

<211>  780
<212>  DNA
<213>  Citrus paradisi

<400>  33
caaaaactac acatgattga tgcagcacaa cgattaggtg tcgcttatca ttttgaaaaa      60

gagattgaag atgaattggg aaaggtatct catgatcttg acagtgatga tctatacgtt     120

gtttctcttc gttttcgact ttttagacag caaggagtta agatttcatg tgatgtgttt     180

gagaagttca aagatgacga aggtaaattc aaggaatcat tgatcaacga tatacgaggc     240

atgtcgagtt tgtacgaggc agcataccta gcaattcggg gggaagacat tttagatgaa     300

gccattgttt tcactaccac tcaccttaag tcagtaatat ctgtatctga tcattctcat     360

gtaaactctg atcttgctga acaaatacgt cattctctgc aaattcctct ccgtaaagcc     420

gcagcaaggt tagaggcaag gtattttttg gatatctatt caagggatga tttgcatgat     480

gaaactttgc tcaagtttgc aaagttagac tttaatatat tacaagcagc acacaagaag     540

gaagcaagta tcatgaccag gtggtggaac gatttaggct tccctaaaaa ggtgccttat     600

gcaagagata gagtagtaga gacatatatt tggatgttgc tgggagtgtc ctatgagccc     660

aatttggcat ttggtagaat ttttgcatcc aaagtggtgt gcataatatc cataatagac     720

gacacatttg atgcttacgg tacttttgaa gagctcacac tttttactga agcagtcaca     780



<210>  34

<211>  28

<212>  DNA

<213>  Artificial sequence

<220>
<223>  Primer deduced (Example 2)

<400>  34
ctgggagtgt cctatgagct caatttgg                                          28


<210>  35

<211>  29

<212>  DNA

<213>  Artificial sequence

<220>
<223>  Primer deduced (Example 2)

<400>  35
gtagaatttt tgcatccaaa gtggtgtgc                                         29

<210>  36

<211>  28

<212>  DNA

<213>  Artificial sequence

<220>
<223>  Primer deduced (Example 2)

<400>  36
cacaccactt tggatgcaaa aattcatc                                          28


<210>  37

<211>  28

<212>  DNA

<213>  Artificial sequence

<220>
<223>  Primer deduced (Example 2)

<400>  37
ccaaattggg ctcataggac actcccag                                          28


<210>  38

<211>  26

<212>  DNA

<213>  Artificial sequence

<220>
<223>  Primer deduced (Example 2)

<400>  38
tagggacgta ttttgaacca aagtac                                            26


<210>  39

<211>  27

<212>  DNA

<213>  Artificial sequence

<220>
<223>  Primer deduced (Example 2)

<400>  39
aaataatgac caaaacaatt tacacgg                                           27


<210>  40

<211>  22

<212>  DNA

<213>  Artificial sequence

<220>
<223>  Primer deduced (Example 2)

<400>  40
gcactttcat gtattctgga ag                                                22


<210>  41

<211>  22

<212>  DNA

<213>  Artificial sequence

<220>
<223>  Primer deduced (Example 2)

<400>  41
gtttgagctc ttcaaagaaa cc                                                22


<210>  42

<211>  31

<212>  DNA

<213>  Artificial sequence

<220>
<223>  Primer deduced (Example 2)

<400>  42
aatgggagtg tattttgagc ctcgatactc c                                      31


<210>  43

<211>  31

<212>  DNA

<213>  Artificial sequence

<220>
<223>  Primer deduced (Example 2)

<400>  43
gatattttcc aaagtaattg caatggcatc c                                      31


<210>  44

<211>  27

<212>  DNA

<213>  Artificial sequence

<220>
<223>  Primer deduced (Example 2)

<400>  44
gttgctaata tcccaccttt tgatagc                                          27


<210>  45

<211>  23

<212>  DNA

<213>  Artificial sequence

<220>
<223>  Primer deduced (Example 2)

<400>  45
aagtgtgcca taggcgtcgt agg                                              23


<210>  46

<211>  27

<212>  DNA

<213>  Artificial sequence

<220>
<223>  Primer deduced (Example 2)

<400>  46
ctgttccgca agcttagggg ttacatg                                          27


<210>  47

<211>  29

<212>  DNA

<213>  Artificial sequence

<220>
<223>  Primer deduced (Example 2)

<400>  47
ctgagctacc aatgacttca ggtgagtgg                                        29


<210>  48

<211>  31

<212>  DNA

<213>  Artificial sequence

<220>

<223>  Primer deduced (Example 2)

<400>  48
caattttgcc atacacatca tagatatcat c                                    31


<210>  49

<211>  28

<212>  DNA

<213>  Artificial sequence

<220>
<223>  Primer deduced (Example 2)

<400>  49
aacagaagtc atggagatca ctttcgtc                                        28


<210>  50

<211>  28

<212>  DNA

<213>  Artificial sequence

<220>
<223>  Primer deduced (Example 2)

<400>  50
cgcaagagat gttttaaagt tcccatcc                                        28


<210>  51

<211>  26

<212>  DNA

<213>  Artificial sequence

<220>
<223>  Primer deduced (Example 2)

<400>  51
tgaacatcag cggaaatttt atagcc                                          26


<210>  52

<211>  24

<212>  DNA

<213>  Artificial sequence

<220>
<223>  Primer deduced (Example 3)

<400>  52

acgatttagg cttccctaaa aagg                                              24

<210>  53

<211>  25

<212>  DNA

<213>  Artificial sequence

<220>
<223>  Primer deduced (Example 3)

<400>  53
tattatggaa tattatgcac accac                                             25

<210>  54

<211>  27

<212>  DNA

<213>  Artificial sequence

<220>
<223>  Primer deduced (Example 3)

<400>  54
aaatgtccgc tcaagttcta gcaacgg                                           27

<210>  55

<211>  26

<212>  DNA

<213>  Artificial sequence

<220>
<223>  Primer deduced (Example 3)

<400>  55
atggcacttc aagattcaga agttcc                                            26

<210>  56

<211>  41

<212>  DNA

<213>  Artificial sequence

<220>
<223>  Primer deduced (Example 4)

<400>  56
gcatgttcca tatgtccgct caagttctag caacggtttc c                           41

<210>  57

<211>  39

<212>  DNA

<213>  Artificial sequence

<220>
<223>  Primer deduced (Example 4)

<400>  57
cgcggatcct cagatggtaa cagggtctct gagcactgc                              39


<210>  58

<211>  37

<212>  DNA

<213>  Artificial sequence

<220>
<223>  Primer deduced (Example 4)

<400>  58
gcatgttcca tatgtcgtct ggagaaacat ttcgtcc                                37


<210>  59

<211>  33

<212>  DNA

<213>  Artificial sequence

<220>
<223>  Primer deduced (Example 4)

<400>  59
cgcggatcct caaaatggaa cgtggtctcc tag                                    33


<210>  60

<211>  39

<212>  DNA

<213>  Artificial sequence

<220>
<223>  Primer deduced (Example 4)

<400>  60
gcatgttcca tatgtctttg gaagtttcag cctctcctg                              39


<210>  61

<211>  42

<212>  DNA

<213>  Artificial sequence

<220>
<223>  Primer deduced (Example 4)

<400>  61
cgcggatcct catatcggca caggattaat aaacaaagaa gc                          42


<210>  62

<211>  30

<212>  DNA

<213>  Artificial sequence

<220>
<223>  Primer deduced (Example 4)

<400>  62
taatggcact tcaagattca gaagttcctc                                        30


<210>  63

<211>  27

<212>  DNA

<213>  Artificial sequence

<220>
<223>  Primer deduced (Example 4)

<400>  63
aaaagggaac aggcttctca agcaatg                                           27


<210>  64

<211>  28

<212>  DNA

<213>  Artificial sequence

<220>
<223>  Primer deduced (Example 4)

<400>  64
atggcacttc aagattcaga agttcctc                                          28


<210>  65

<211>  31

<212>  DNA

<213>  Artificial sequence

<220>
<223>  Primer deduced (Example 4)

<400>  65
gatcaaaagg gaacaggctt ctcaagcaat g                                    31

## Description

This application claims the benefit of U.S. Provisional Application No. 60/415,765, filed October 4, 2002, and the contents of which are incorporated by reference herein. This application also claims the benefit of priority of International Application, PCT/IB02/05070, filed December 2, 2002, the contents of which are incorporated by reference herein.

The present invention relates to sesquiterpene synthases and methods of their production and use. In one embodiment, the invention provides nucleic acids comprising a nucleotide sequence as described herein that encodes for at least one sesquiterpene synthase. In a further embodiment, the invention also provides for sesquiterpene synthases and methods of making and using these enzymes. For example, sesquiterpene synthases of the invention may be used to convert farnesyl-pyrophosphate to various oxygenated and aliphatic sesquiterpenes including valencene, bicyclo-germacrene, cubebol and delta-cadine.

### Background of the Invention

Terpene compounds represent a wide range of natural molecules with a large diversity of structure. The plant kingdom contains the highest diversity of monoterpenes and sesquiterpenes. Often they play a role in defense of the plants against pathogens, insects and herbivored and for attraction of pollinating insect.

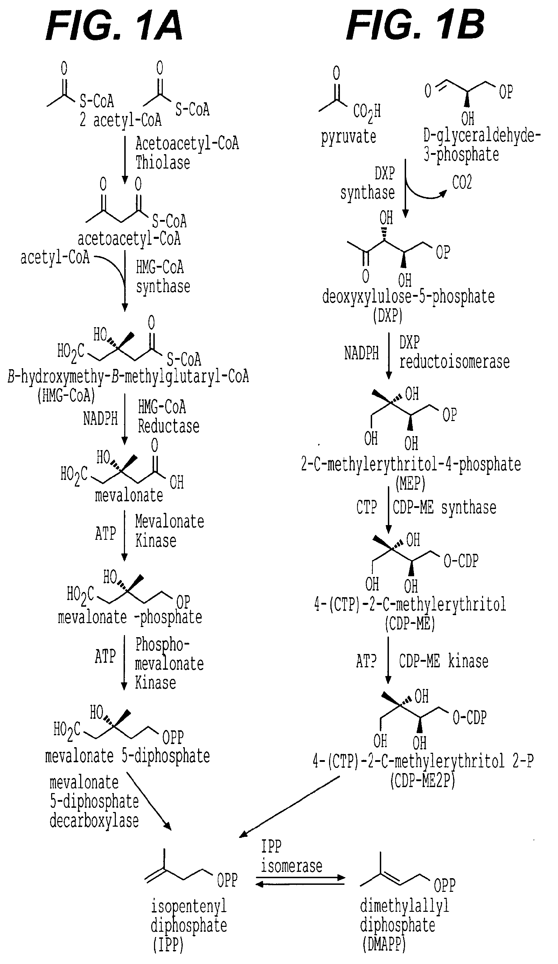

The biosynthesis of terpenes has been extensively studied in many organisms. The common precursor to terpenes is isopentenyl pyrophosphate (IPP) and many of the enzymes catalyzing the steps leading to IPP have been characterized. Two distinct pathways for IPP biosynthesis are currently known (Figure 1). The mevalonate pathway is found in the plants cytosol and in yeast and the non-mevalonate pathway (or deoxyxylulose-5-phosphate (DXP) pathway is found in the plant plastids and in E. coli.

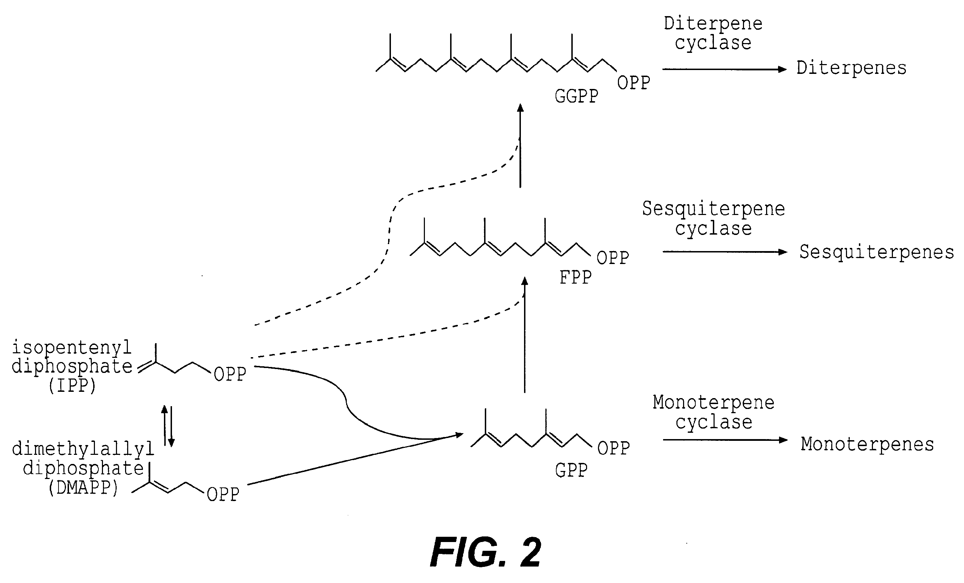

For example, the IPP is isomerized to dimethylallyl diphosphate by the IPP isomerase and these two C5 compounds can be condensed by prenyl transferases to form the acyclic pyrophosphate terpene precursors for each class of terpenes, i.e. geranyl-pyrophosphate (GPP) for the monoterpenes, farnesyl-pyrophosphate (FPP) for the sesquiterpenes, geranylgeranyl-pyrophosphate (GGPP) for the diterpenes (Figure 2). The enzymes catalyzing the cyclisation step of the acyclic precursors are named terpene cyclases or terpene synthases, which are referred to as terpene synthases herein.

These enzymes may be able to catalyze complex multiple step cyclization to form the carbon skeleton of a terpene or sesquiterpene compound. For example, the initial step of the catalyzed cyclisation may be the ionization of the diphosphate group to form a allylic cation. The substrate then undergoes isomerizations and rearrangements which can be controlled by the enzyme active site. The product may, for example, be acyclic, mono-, di or tri-cyclic. A proton may then be released from the carbocation or the carbocation reacts with a water molecule and the terpene hydrocarbon or alcohol is released. Some terpene synthases produce a single product, but many produce multiple products.

A large diversity of terpene structures and sesquiterpene synthases are found in nature. Several sesquiterpene synthase encoding cDNA or genes have been cloned and characterized from different plant sources, e.g, 5-epi-aristolochene synthases form Nicotiana tabacum (Facchini, P.J. and Chappell, J. (1992) Proc. Natl. Acad. Sci. U.S.A. 89, 11088-11092.) and from Capsicum annum (Back, K., et al. (1998) Plant Cell Physiol. 39 (9), 899-904.), a vetispiradiene synthase from Hyoscyamus muticus (Back, K. and Chappell, J. (1995) J. Biol. Chem. 270 (13), 7375-7381.), a (E)-β-farnesene synthase from Mentha piperita (Crock, J., et al. (1997) Proc. Natl. Acad. Sci. U.S.A. 94 (24), 12833-12838.), a δ-selinene synthase and a γ-humulene synthase from Abies grandis (Steele, C.L., et al. (1998) J. Biol. Chem. 273 (4), 2078-2089.), δ-cadinene synthases from Gossypium arboreum (Chen, X.Y., et al. (1995) Arch. Biochem. Biophys. 324 (2), 255-266; Chen, X.Y., et al. (1996) J. Nat Prod. 59, 944-951.), a E-α-bisabolene synthase from Abies grandis (Bohlmann, J., et al. (1998) Proc. Natl. Acad. Sci. U.S.A. 95 (12), 6756-6761.), a germacrene C synthase from Lycopersicon esculentum (Colby, S.M., et al. (1998) Proc. Natl. Acad. Sci. U.S.A. 95 (5), 2216-2221.), an epi-cedrol synthase and an amorpha-4,11-diene synthase from Artemisia annua (Mercke, P., et al. (1999) Arch. Biochem. Biophys. 369 (2), 213-222; Mercke, P., et al. (2000) Arch. Biochem. Biophys. 381 (2), 173-180.), and germacrene A synthases from Lactuca sativa, from Cichorium intybus and from Solidago canadensis (Bennett, M.H., et al. (2002) Phytochem. 60, 255-261; Bouwmeester, H.J., et al. (2002) Plant Physiol. 129 (1), 134-144; Prosser I, et al. (2002) Phytochem. 60, 691-702).

Many sesquiterpene compounds are used in perfumery (e.g. patchoulol, nootkatone, santalol, vetivone, sinensal) and many are extracted from plants. As a result, their availability and prices may be subject to fluctuation related to the availability of the plants and the stability of the producing countries. The availability of a plant-independent system for production of sesquiterpenes may therefore be of interest. Due to the structural complexity of some sesquiterpenes, their chemical synthesis at an acceptable cost may not always be feasible.

### Summary of the Invention

In one embodiment, the invention relates to isolated nucleic acids that encode sesquiterpene synthases. As used herein, a sesquiterpene synthase may also be referred to by at least one compound produced by the enzyme upon contact with an acyclic pyrophosphate terpene precursor such as farnesyl-pyrophosphate. For example, a sesquiterpene synthase capable of producing bicylogermacrene as one of its products may be referred to as bicylogermacrene synthase. Using this convention, examples of nucleic acids of the invention include cDNAs encoding cubebol synthase (GFTpsC) (SEQ ID NO:1); δ-cadine synthase (GFTpsE) (SEQ ID NO:2); bicylogermacrene synthase (GFTpsB) (SEQ ID NO:3 ); valencene synthase (GFTpsDI & GFTpsD2) (SEQ ID NO:4 & SEQ ID NO:5); and.

In one embodiment, the invention provides an isolated nucleic acid selected from: (a) a nucleic acid comprising the nucleotide sequence substantially as set out in SEQ ID NO:6, SEQ ID NO:7, SEQ ID NO:8, SEQ ID NO:9, or SEQ ID NO:10; (b) a nucleic acid encoding the polypeptide substantially set out in SEQ ID NO:1, SEQ ID NO:2, SEQ ID NO:3, SEQ ID NO:4, or SEQ ID NO:5; and (c) a nucleic acid that hybridizes to the nucleic acid of (a) or (b) under low stringency conditions, wherein the polypetide encoded by said nucleic acid is a sesquiterpene synthase. In one embodiment, the defined conditions are moderate stringency conditions and in a further embodiment high stringency conditions. Other embodiments include: a polypeptide encoded by a nucleic acid of the invention; a host cell comprising a nucleic acid of the invention; a non-human organism modified to harbor a nucleic acid of the invention; and methods of producing a polypeptide comprising culturing host cells of the invention.

In another embodiment, the invention provides an isolated polypeptide comprising an amino acid sequence substantially as set out in SEQ ID NO:1, SEQ ID NO:2, SEQ ID NO:3, SEQ ID NO:4, or SEQ ID NO:5.

In a further embodiment, the invention provides a vector comprising at least one nucleic acid chosen from (a) a nucleic acid comprising the nucleotide sequence substantially as set out in SEQ ID NO:6, SEQ ID NO:7, SEQ ID NO:8, SEQ ID NO:9, or SEQ ID NO:10; (b) a nucleic acid encoding the polypeptide substantially set out in SEQ ID NO:1, SEQ ID NO:2, SEQ ID NO:3, SEQ ID NO:4, or SEQ ID NO:5; and (c) a nucleic acid that hybridizes to the nucleic acid of (a) or (b) under low stringency conditions, wherein the polypetide encoded by said nucleic acid is a sesquiterpene synthase. Other embodiments include, methods of making a recombinant host cell comprising introducing a vector of the invention into a host cell.

In one embodiment, the invention provides a method of making at least one sesquiterpene synthase comprising culturing a host modified to contain at least one nucleic acid sequence under conditions conducive to the production of said at least one sesquiterpene synthase. In one embodiment, the at least one nucleic acid is chosen from (a) a nucleic acid comprising the nucleotide sequence substantially as set out in SEQ ID NO:6, SEQ ID NO:7, SEQ ID NO:8, SEQ ID NO:9, or SEQ ID NO:10; (b) a nucleic acid encoding the polypeptide substantially set out in SEQ ID NO:1, SEQ ID NO:2, SEQ ID NO:3, SEQ ID NO:4, or SEQ ID NO:5; and (c) a nucleic acid that hybridizes to the nucleic acid of (a) or (b) under low stringency conditions, wherein the polypetide encoded by said nucleic acid is a sesquiterpene synthase. The host may be chosen from, for example, plants, microorganisms, bacterial cells, yeast cells, plant cells, and animal cells.

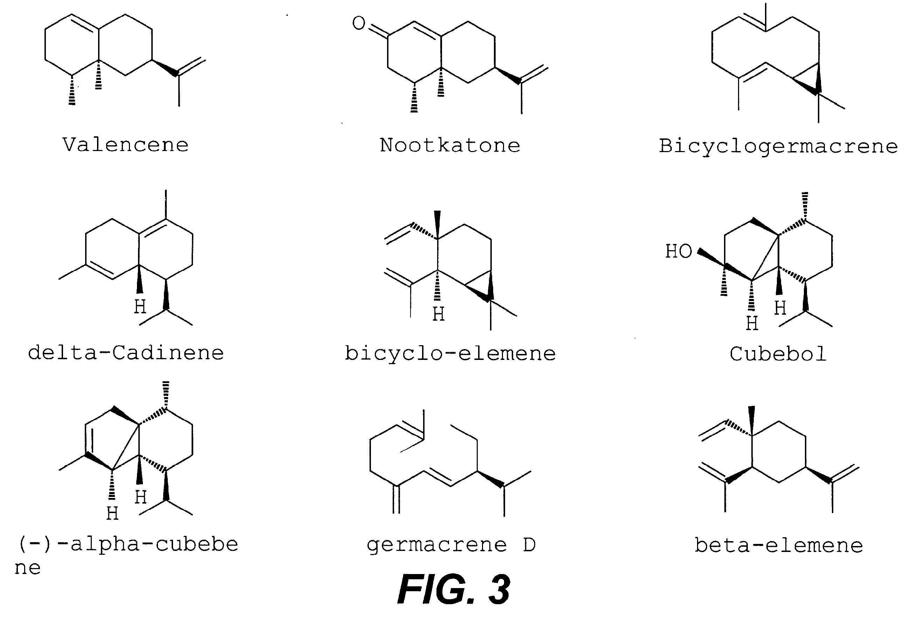

In another embodiment the invention provides a method of making at least one terpenoid comprising 1) contacting at least one acyclic pyrophosphate terpene precursor with at least one polypeptide encoded by a nucleic acid. In one embodiment, the nucleic acid is chosen from (a) a nucleic acid comprising the nucleotide sequence substantially as set out in SEQ ID NO:6, SEQ ID NO:7, SEQ ID NO:8, SEQ ID NO:9, or SEQ ID NO:10; (b) a nucleic acid encoding the polypeptide substantially set out in SEQ ID NO:1, SEQ ID NO:2, SEQ ID NO:3, SEQ ID NO:4, or SEQ ID NO:5; and (c) a nucleic acid that hybridizes to the nucleic acid of (a) or (b) under low stringency conditions, wherein the polypetide encoded by said nucleic acid is a sesquiterpene synthase, 2) isolating at least one terpenoid produced in (1). In one embodiment, the at least one terpenoid is chosen from sesquiterpenes. In a further embodiment, the at least one acyclic pyrophosphate terpene precursor is farnesyl-pyrophosphate. The sesquiterpenes produced by the methods of the invention include, but are not limited to, bicylogermacrene, cubebol, valencene, α-cubebene, germacrene D, and δ-cadinene (Figure 3).

It is to be understood that both the foregoing general description and the following detailed description are exemplary and explanatory only and are not restrictive of the invention as claimed. Reference will now be made in detail to exemplary embodiments of the present invention.

### Brief Description of the Figures

Figure 1 : Example pathways for biosynthesis of isopentenyl pyrophosphate (Mevalonate pathway (A) and deoxyxylulose pathway (B)).
Figure 2: Example terpene biosynthesis from isopentenyl diphosphoate.
Figure 3: Structure of example sesquiterpene compounds.

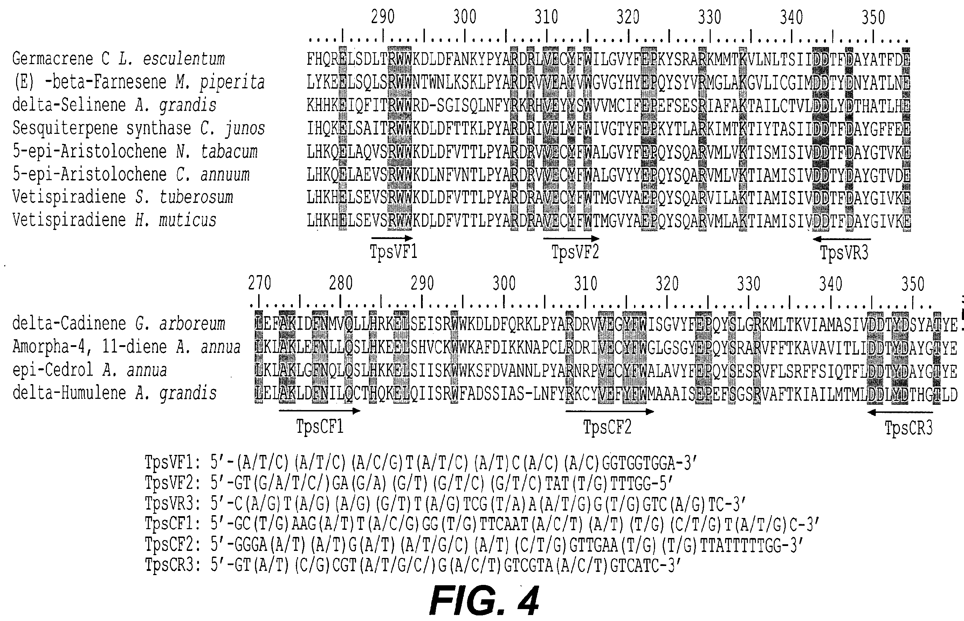

Figure 4: Central part of the alignments of the amino acid sequences of two groups of sesquitepene synthase (Germacrene C *L. esculentum* δ-cadine synthase (SEQ ID NO: 11); (E)-beta-Famesene M. *piperita* (SEQ ID NO: 12); delta-Selinene *A. grandis* (SEQ ID NO: 13); Sesquiterpene synthase *C. junos* (SEQ ID NO:14); 5-epi-Aristolochene *N*. *tabacum* (SEQ ID NO:15); 5-epi-Aristolochene C. *annuum* (SEQ ID NO:16); Vetispiradiene *S.tuberosum* (SEQ ID NO:17); Vetispiradiene *H. muticus* (SEQ ID NO:18); delta-Cadinene *G. arboreum* (SEQ ID NO:19); Amorpha-4,11-*diene A. annua* (SEQ ID NO:20); epi-Cedrol *A. annua* (SEQ ID NO:21); delta-Humulene *A. grandis* (SEQ ID NO:22)) and the sequence of the deduced degenerate primers (TpsVFI (SEQ ID NO:23); TpsVF2 (SEQ ID NO:24); TpsVR3 (SEQ ID NO:25); TpsCFI (SEQ ID NO:26); TpsCF2 (SEQ ID NO:27); TpsCR3 (SEQ ID NO:28)). The arrows below each alignment show the regions of the alignment used to design the degenerate primers and their orientation.
Figure 5: Alignment of the amino acid sequences deduced from the amplification products obtained by RT-PCR on grapefruit total RNA (GFTpsA (SEQ ID NO:29); GFTpsB (SEQ ID NO:30); and GFTpsC (SEQ ID NO:31)).
Figure 6: Amino acid sequence alignment of the sesquiterpene synthases GFTpsA (partial clone) (SEQ ID NO:32), GFTpsB (SEQ ID NO:3), GFTbsC (SEQ ID NO:1), GFTpsDI (SEQ ID NO:4), GFTpsD2 (SEQ ID NO:5), and GFTpsE (SEQ ID NO:2) from C. paradisi.
Figure 7: GC profiles of sesquiterpenes produced by recombinant grapefruit sesquiterpene synthases.

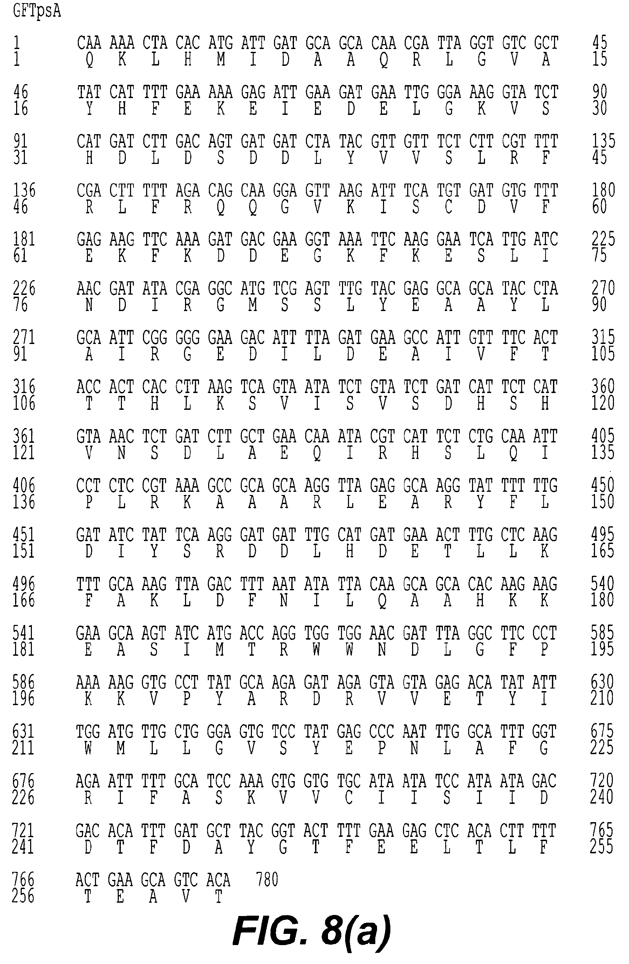

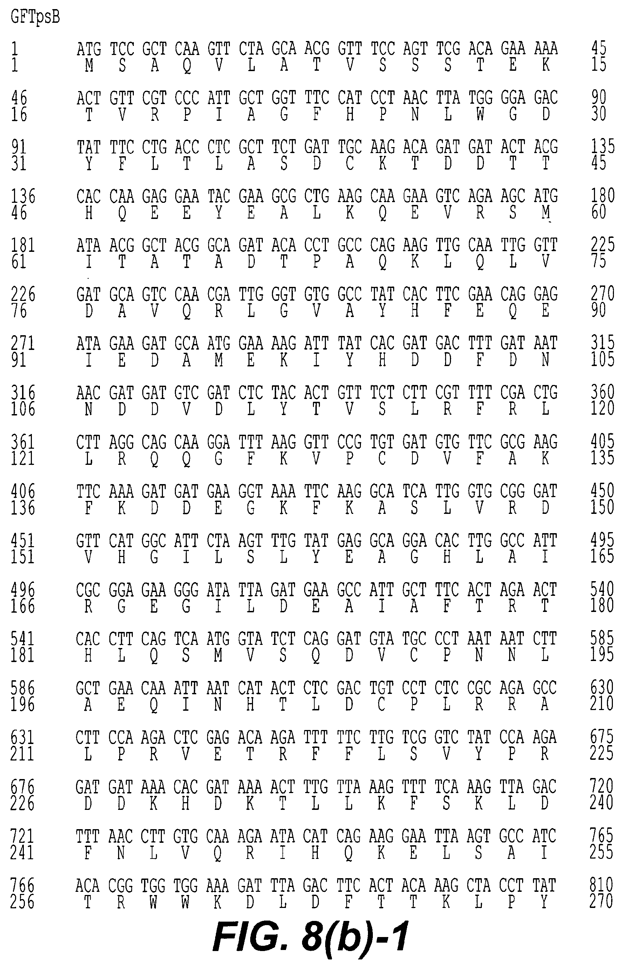

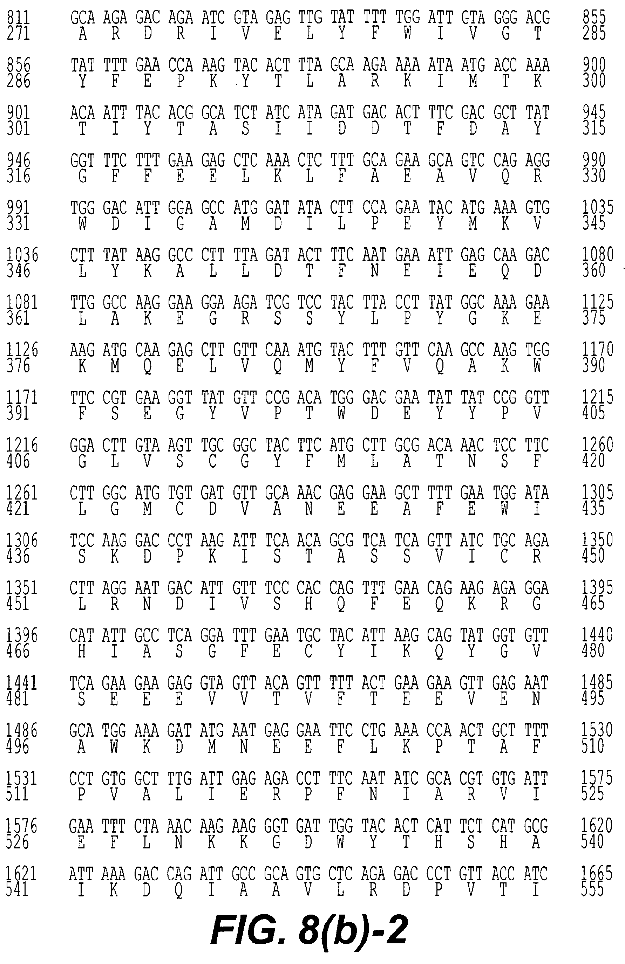

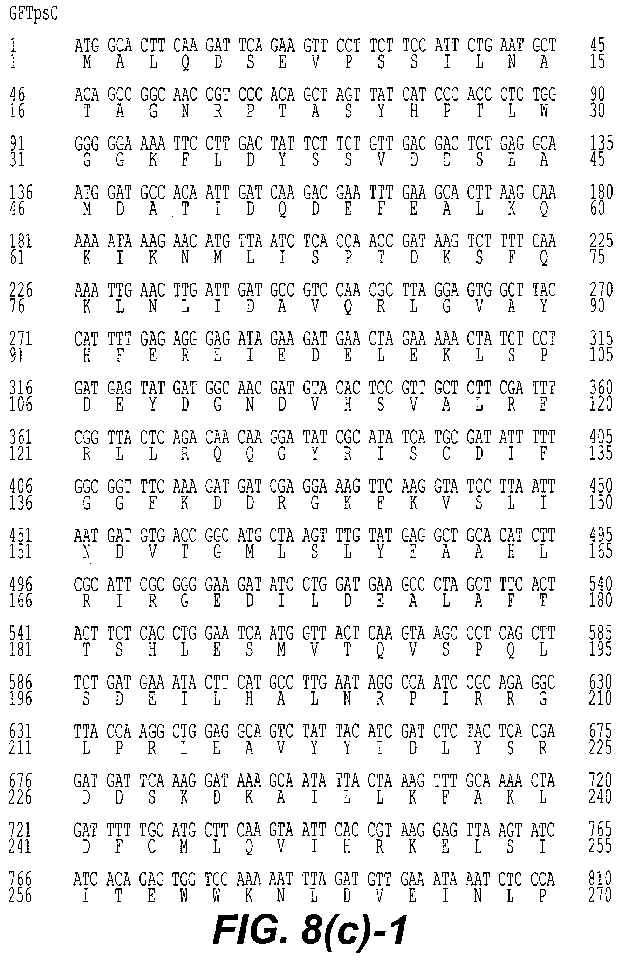

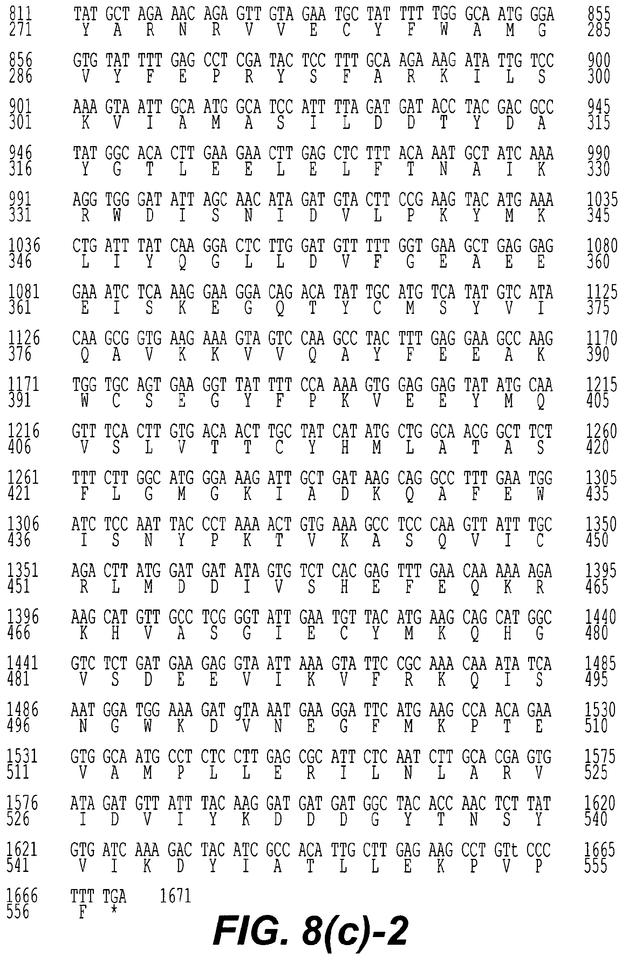

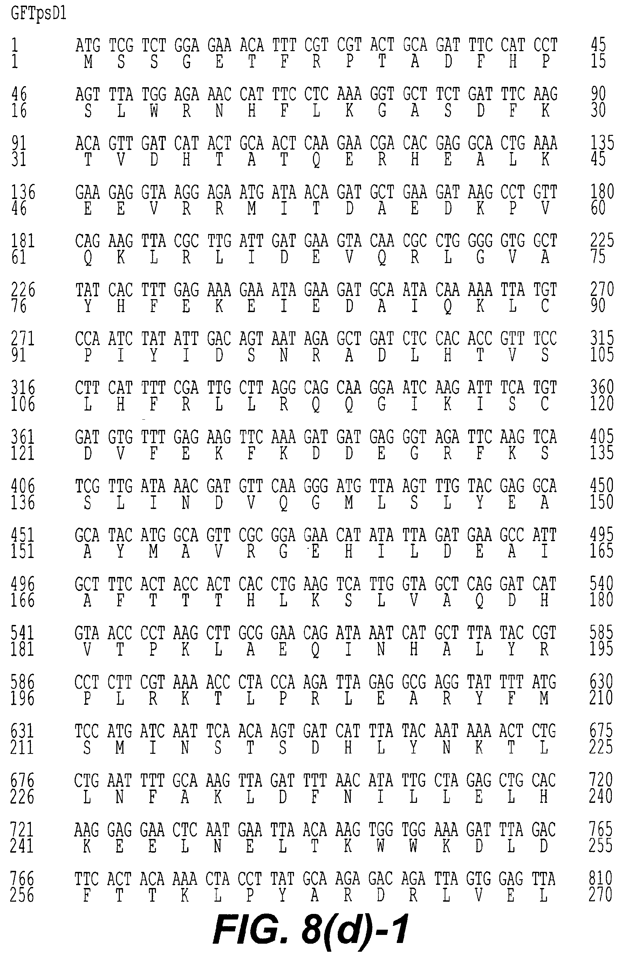

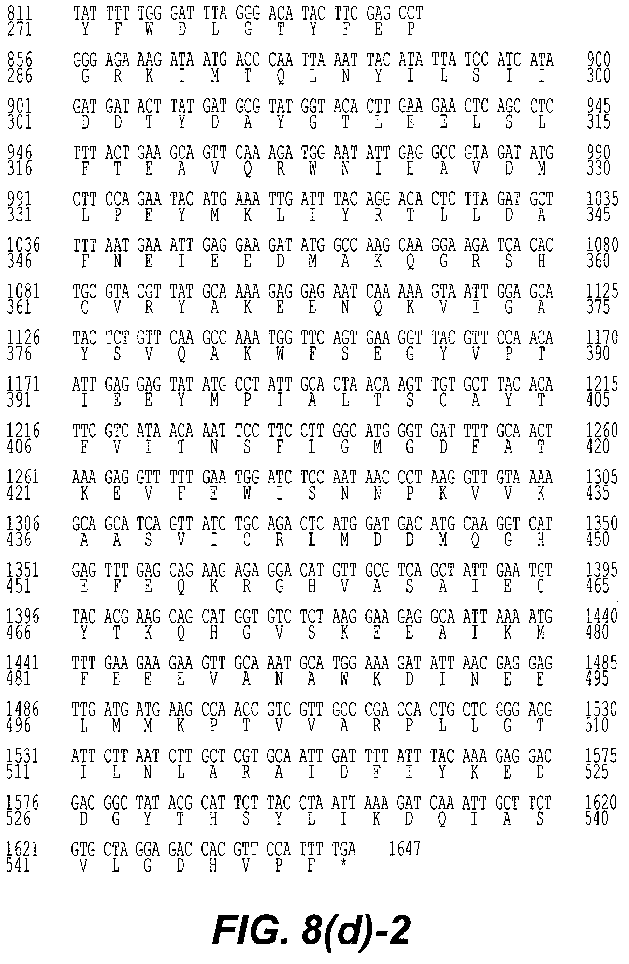

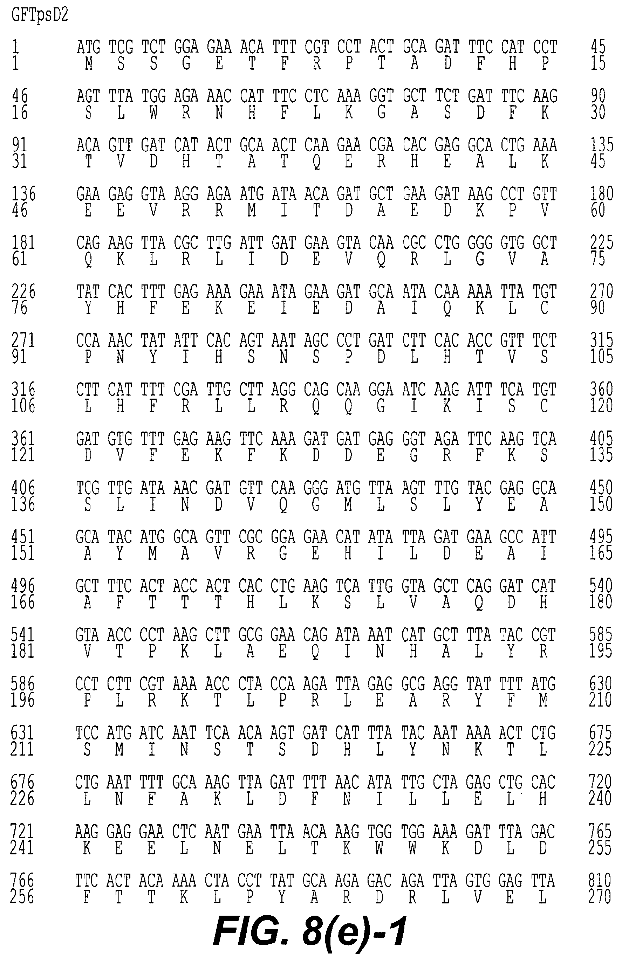

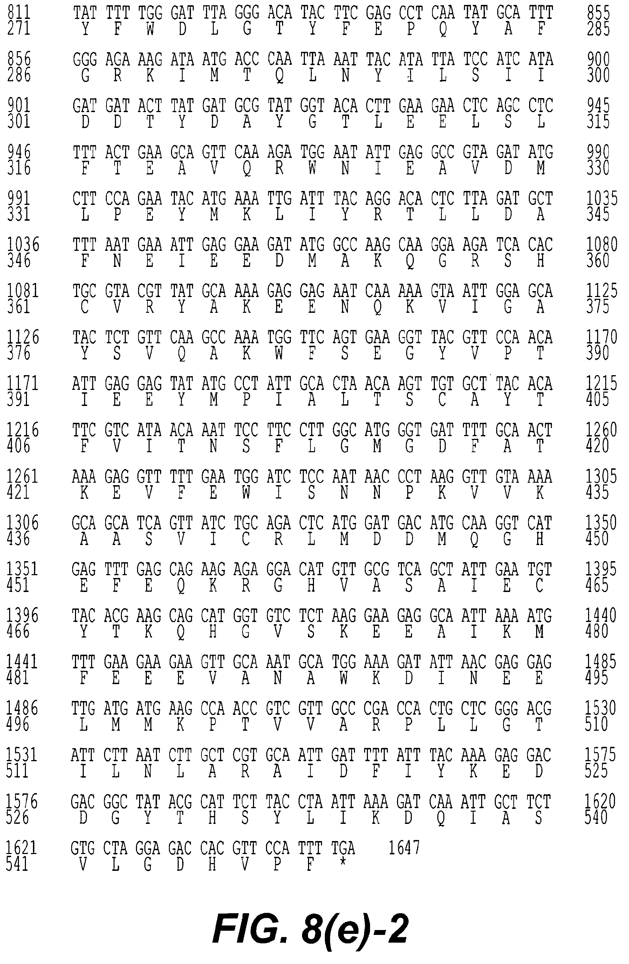

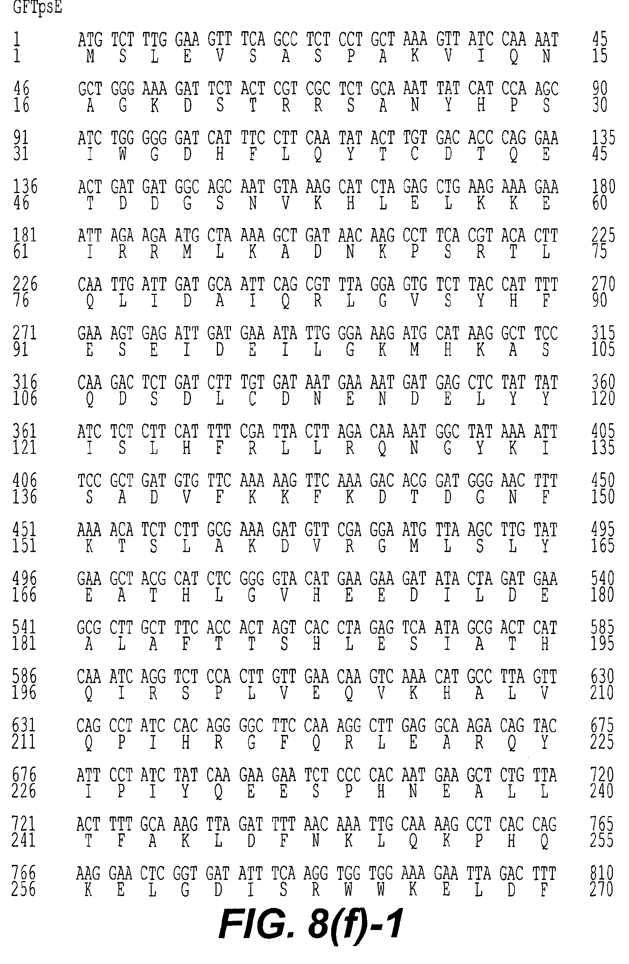

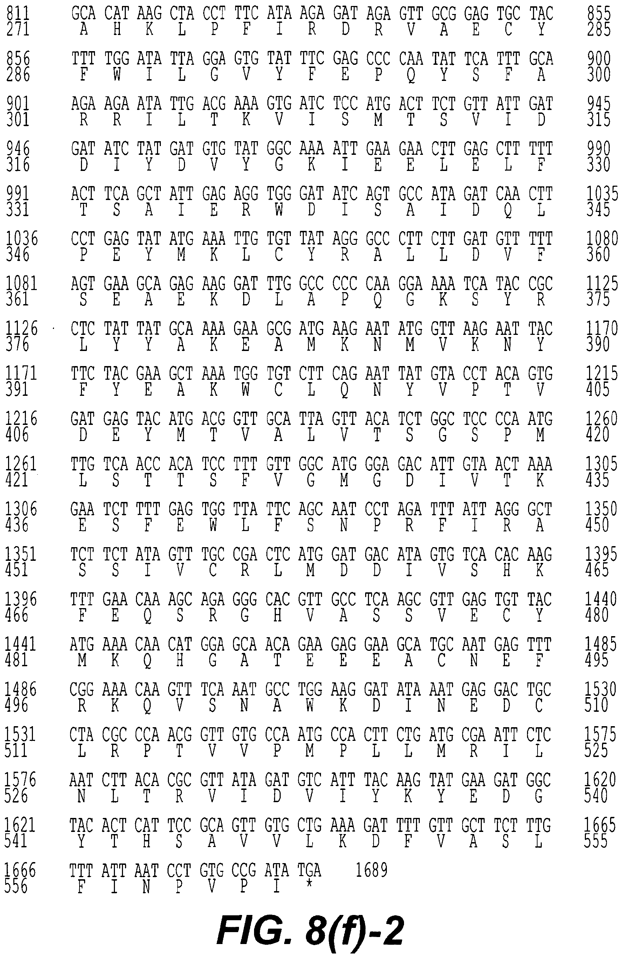

Figure 8: The amino acid and nucleotide sequences of (a) GFTpsA (SEQ ID NO:32 and SEQ ID NO:33), (b) GFTpsB (SEQ ID NO:3 and SEQ ID NO:8), (c) GFTbsC (SEQ ID NO:1 and SEQ ID NO:6), (d) GFTpsDI (SEQ ID NO:4 and SEQ ID NO:9), (e) GFTpsD2 (SEQ ID NO:5 and SEQ ID NO:10), and (f) GFTpsE (SEQ ID NO:2 and SEQ ID NO:7).

### Description of the Invention

A terpene is an unsaturated hydrocarbon based on an isoprene unit (C5H8) which may be acyclic or cyclic. Terpene derivatives, include but are not limited to camphor, menthol, terpineol, and borneol, geraniol. Terpenes or Terpenoid, as used herein includes terpenes and terpene derivatives, including compounds that have undergone one or more steps of functionalization such as hydroxylations, isomerizations, oxido-reductions or acylations. As used herein, a sesquiterpene is terpene based on a C15 structure and includes sesquiterpenes and sesquiterpenes derivatives, including compounds that have undergone one or more steps of functionalization such as hydroxylations, isomerizations, oxido-reductions or acylations.

As used herein, a derivative is any compound obtained from a known or hypothetical compound and containing essential elements of the parent substance.

As used herein, sesquiterpene synthase is any enzyme that catalyzes the synthesis of a sesquiterpene.

The phrase "identical," "substantially identical," or "substantially as set out," means that a relevant sequence is at least 70%, 75%, 80%, 85%, 90%, 92%, 95%, 96%, 97%, 98%, or 99% identical to a given sequence. By way of example, such sequences may be allelic variants, sequences derived from various species, or they may be derived from the given sequence by truncation, deletion, amino acid substitution or addition. For polypeptides, the length of comparison sequences will generally be at least 20, 30, 50, 100 or more amino acids. For nucleic acids, the length of comparison sequences will generally be at least 50, 100, 150, 300, or more nucleotides. Percent identity between two sequences is determined by standard alignment algorithms such as, for example, Basic Local Alignment Tool (BLAST) described in Altschul et al. (1990) J. Mol. Biol., 215:403-410, the algorithm of Needleman et al. (1970) J. Mol. Biol., 48:444-453, or the algorithm of Meyers et al. (1988) Comput. Appl. Biosci., 4:11-17.

The invention thus provides, in one embodiment, an isolated nucleic acid selected from: (a) a nucleic acid comprising the nucleotide sequence sequence substantially as set out in SEQ ID NO:6, SEQ ID NO:7, SEQ ID NO:8, SEQ ID NO:9, or SEQ ID NO:10; (b) a nucleic acid encoding the polypeptide substantially set out in SEQ ID NO:1, SEQ ID NO:2, SEQ ID NO:3, SEQ ID NO:4, or SEQ ID NO:5; and (c) a nucleic acid that hybridizes to the nucleic acid of (a) or (b) under low stringency conditions, wherein the polypetide encoded by said nucleic acid is a sesquiterpene synthase. In one embodiment, the defined conditions are moderate stringency conditions and in a further embodiment high stringency conditions.

As used herein, one determines whether a polypeptide encoded by a nucleic acid of the invention is a sesquiterpene synthase by the enzyme characterization assay described in the examples herein.

As used herein, the term hybridization or hybridizes under certain conditions is intended to describe conditions for hybridization and washes under which nucleotide sequences that are significantly identical or homologous to each other remain bound to each other. The conditions may be such that sequences, which are at least about 70%, such as at least about 80%, and such as at least about 85-90% identical, remain bound to each other. Definitions of low stringency, moderate, and high stringency hybridization conditions are provided herein.

Appropriate hybridization conditions can be selected by those skilled in the art with minimal experimentation as exemplified in Ausubel et al. (1995), Current Protocols in Molecular Biology, John Wiley & Sons, sections 2, 4, and 6. Additionally, stringency conditions are described in Sambrook et al. (1989) Molecular Cloning: A Laboratory Manual, 2nd ed., Cold Spring Harbor Press, chapters 7, 9, and 11. As used herein, defined conditions of low stringency are as follows. Filters containing DNA are pretreated for 6 h at 40°C. in a solution containing 35% formamide, 5x SSC, 50 mM Tris-HCl (pH 7.5), 5 mM EDTA, 0.1% PVP, 0.1% Ficoll, 1% BSA, and 500 µg/ml denatured salmon sperm DNA. Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20x106 32P-labeled probe is used. Filters are incubated in hybridization mixture for 18-20 h at 40°C, and then washed for 1.5 h at 55°C. In a solution containing 2x SSC, 25 mM Tris-HCl (pH 7.4), 5 mM EDTA, and 0.1 % SDS. The wash solution is replaced with fresh solution and incubated an additional 1.5 h at 60°C. Filters are blotted dry and exposed for autoradiography.

As used herein, defined conditions of moderate stringency are as follows. Filters containing DNA are pretreated for 7 h at 50°C. in a solution containing 35% formamide, 5x SSC, 50 mM Tris-HCl (pH 7.5), 5 mM EDTA, 0.1% PVP, 0.1% Ficoll, 1% BSA, and 500 µg/ml denatured salmon sperm DNA. Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20x106 32P-labeled probe is used. Filters are incubated in hybridization mixture for 30 h at 50°C, and then washed for 1.5 h at 55°C. In a solution containing 2x SSC, 25 mM Tris-HCl (pH 7.4), 5 mM EDTA, and 0.1% SDS. The wash solution is replaced with fresh solution and incubated an additional 1.5 h at 60°C. Filters are blotted dry and exposed for autoradiography.

As used herein, defined conditions of high stringency are as follows. Prehybridization of filters containing DNA is carried out for 8 h to overnight at 65°C in buffer composed of 6x SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 h at 65°C in the prehybridization mixture containing 100 µg /ml denatured salmon sperm DNA and 5-20x106 cpm of 32P-labeled probe. Washing of filters is done at 37°C for 1 h in a solution containing 2x SSC, 0.01% PVP, 0.01% Ficoll, and 0.01% BSA. This is followed by a wash in 0.1x SSC at 50°C for 45 minutes.

Other conditions of low, moderate, and high stringency well known in the art (e.g., as employed for cross-species hybridizations) may be used if the above conditions are inappropriate (e.g., as employed for cross-species hybridizations).

In one embodiment, a nucleic acid and/or polypeptide of the invention is isolated from a citrus, such as, for example a grapefruit or an orange. In a particular embodiment, the invention relates to certain isolated nucleotide sequences including those that are substantially free from contaminating endogenous material. The terms "nucleic acid" or "nucleic acid molecule" refer to a deoxyribonucleotide or ribonucleotide polymer in either single-or double-stranded form, and unless otherwise limited, would encompass known analogs of natural nucleotides that can function in a similar manner as naturally occurring nucleotides. A "nucleotide sequence" also refers to a polynucleotide molecule or oligonucleotide molecule in the form of a separate fragment or as a component of a larger nucleic acid. The nucleotide sequence or molecule may also be referred to as a "nucleotide probe." Some of the nucleic acid molecules of the invention are derived from DNA or RNA isolated at least once in substantially pure form and in a quantity or concentration enabling identification, manipulation, and recovery of its component nucleotide sequence by standard biochemical methods. Examples of such methods, including methods for PCR protocols that may be used herein, are disclosed in Sambrook et al., Molecular Cloning: A Laboratory Manual, 2nd ed., Cold Spring Harbor Laboratory, Cold Spring Harbor, NY (1989), Current Protocols in Molecular Biology edited by F.A. Ausubel et al., John Wiley and Sons, Inc. (1987), and Innis, M. et al., eds., PCR Protocols: A Guide to Methods and Applications, Academic Press (1990).

As described herein, the nucleic acid molecules of the invention include DNA in both single-stranded and double-stranded form, as well as the RNA complement thereof. DNA includes, for example, cDNA, genomic DNA, chemically synthesized DNA, DNA amplified by PCR, and combinations thereof. Genomic DNA, including translated, non-translated and control regions, may be isolated by conventional techniques, e.g., using any one of the cDNAs of the invention, or suitable fragments thereof, as a probe, to identify a piece of genomic DNA which can then be cloned using methods commonly known in the art. In general, nucleic acid molecules within the scope of the invention include sequences that hybridize to sequences of the invention under hybridization and wash conditions described above and of 5°, 10°, 15°, 20°, 25°, or 30° below the melting temperature of the DNA duplex of sequences of the invention, including any range of conditions subsumed within these ranges.

In another embodiment, the nucleic acids of the invention comprises a sequence substantially as set out in SEQ ID NO:6, SEQ ID NO:7, SEQ ID NO:8, SEQ ID NO:9, or SEQ ID NO:10. In one embodiment, the nucleic acids are at least 85%, at least 90%, or at least 95% identical to nucleotides SEQ ID NO:6, SEQ ID NO:7, SEQ ID NO:8, SEQ ID NO:9, or SEQ ID NO:10. In one embodiment, the nucleic acid comprises the nucleotide sequence SEQ ID NO:6, SEQ ID NO:7, SEQ ID NO:8, SEQ ID NO:9, or SEQ ID NO:10. In a further embodiment, the nucleic acid encodes a protein that is a sesquiterpenes synthase, as demonstrated, for example, in the enzyme assay described in the examples. Nucleic acids comprising regions conserved among different species, are also provided.

In yet another embodiment, the nucleic acid comprises a contiguous stretch of at least 50, 100, 250, 500, 750 contiguous nucleotides of SEQ ID NO:6, SEQ ID NO:7, SEQ ID NO:8, SEQ ID NO:9, or SEQ ID NO:10. Such contiguous fragments of these nucleotides may also contain at least one mutation so long as the mutant sequence retains the functionality of the original sequence and the capacity to hybridize to these nucleotides under low or high stringency conditions, such as for example, moderate or high stringency conditions. Such a fragment can be derived, for example, from nucleotide (nt) 200 to nt 1600, from nt 800 to nt 1600, from nt 1000 to nt 1600, from nt 200 to nt 1000, from nt 200 to nt 800, from nt 400 to nt 1600, or from nt 400 to nt 1000 of SEQ ID NO:6, SEQ ID NO:7, SEQ ID NO:8, SEQ ID NO:9, or SEQ ID NO:10.

As described above, polypeptides encoded by the nucleic acids of the invention are encompassed by the invention. The isolated nucleic acids of the invention may be selected from a nucleic acid encoding the polypeptide substantially set out in SEQ ID NO:1, SEQ ID NO:2, SEQ ID NO:3, SEQ ID NO:4, or SEQ ID NO:5. In one embodiment, the polypeptides are at least 85%, at least 90%, or at least 95% identical to SEQ ID NO:1, SEQ ID NO:2, SEQ ID NO:3, SEQ ID NO:4, or SEQ ID NO:5.

In one embodiment, a polypeptide of the invention comprises an amino acid sequence as set out in SEQ ID NO:1, SEQ ID NO:2, SEQ ID NO:3, SEQ ID NO:4, or SEQ ID NO:5. In another embodiment, the polypeptide comprises an amino acid sequence substantially as set out in of SEQ ID NO:1, SEQ ID NO:2, SEQ ID NO:3, SEQ ID NO:4, or SEQ ID NO:5. In yet another embodiment, the polypeptide comprises an amino acid sequence that is at least 85% identical, at least 90% or at least 95% identical to of SEQ ID NO:1, SEQ ID NO:2, SEQ ID NO:3, SEQ ID NO:4, or SEQ ID NO:5. In one embodiment, the polypeptide is a sesquiterpene synthase, as demonstrated, for example, in the enzyme assay described below.

Due to the degeneracy of the genetic code wherein more than one codon can encode the same amino acid, multiple DNA sequences can code for the same polypeptide. Such variant DNA sequences can result from genetic drift or artificial manipulation (e.g., occurring during PCR amplification or as the product of deliberate mutagenesis of a native sequence). The present invention thus encompasses any nucleic acid capable of encoding a protein derived from the SEQ ID NO:6, SEQ ID NO:7, SEQ ID NO:8, SEQ ID NO:9, or SEQ ID NO: 10 or variants thereof.

Deliberate mutagenesis of a native sequence can be carried out using numerous techniques well known in the art. For example, oligonucleotide-directed site-specific mutagenesis procedures can be employed, particularly where it is desired to mutate a gene such that predetermined restriction nucleotides or codons are altered by substitution, deletion or insertion. Exemplary methods of making such alterations are disclosed by Walder et al. (Gene 42:133, 1986); Bauer et al. (Gene 37:73, 1985); Craik (BioTechniques, January 12-19, 1985); Smith et al. (Genetic Engineering: Principles and Methods, Plenum Press, 1981); Kunkel (Proc. Natl. Acad. Sci. USA 82:488, 1985); Kunkel et al. (Methods in Enzymol. 154:367, 1987); and U.S. Patent Nos. 4,518,584 and 4,737,462.

In one embodiment, the invention provides for isolated polypeptides. As used herein, the term "polypeptides" refers to a genus of polypeptide or peptide fragments that encompass the amino acid sequences identified herein, as well as smaller fragments. Alternatively, a polypeptide may be defined in terms of its antigenic relatedness to any peptide encoded by the nucleic acid sequences of the invention. Thus, in one embodiment, a polypeptide within the scope of the invention is defined as an amino acid sequence comprising a linear or 3-dimensional epitope shared with any peptide encoded by the nucleic acid sequences of the invention. Alternatively, a polypeptide within the scope of the invention is recognized by an antibody that specifically recognizes any peptide encoded by the nucleic acid sequences of the invention. Antibodies are defined to be specifically binding if they bind polypeptides of the invention with a Kₐ of greater than or equal to about 10⁷ M⁻¹, such as greater than or equal to 10⁸ M⁻¹.

A polypeptide "variant" as referred to herein means a polypeptide substantially homologous to a native polypeptide, but which has an amino acid sequence different from that encoded by any of the nucleic acid sequences of the invention because of one or more deletions, insertions or substitutions.

Variants can comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gin and Asn. See Zubay, Biochemistry, Addison-Wesley Pub. Co., (1983). The effects of such substitutions can be calculated using substitution score matrices such a PAM-120, PAM-200, and PAM-250 as discussed in Altschul, (J. Mol. Biol. 219:555-65, 1991). Other such conservative substitutions, for example, substitutions of entire regions having similar hydrophσbicity characteristics, are well known.

Naturally-occurring peptide variants are also encompassed by the invention. Examples of such variants are proteins that result from alternate mRNA splicing events or from proteolytic cleavage of the polypeptides described herein. Variations attributable to proteolysis include, for example, differences in the N- or C-termini upon expression in different types of host cells, due to proteolytic removal of one or more terminal amino acids from the polypeptides encoded by the sequences of the invention.

Variants of the sesquiterpenes synthases of the invention may be used to attain desired enhanced or reduced enzymatic activity, modified regiochemistry or stereochemistry, or altered substrate utilization or product distribution. A variant or site direct mutant may be made by any methods known in the art.

As stated above, the invention provides recombinant and non-recombinant, isolated and purified polypeptides, such as from citrus plants. Variants and derivatives of native polypeptides can be obtained by isolating naturally-occurring variants, or the nucleotide sequence of variants, of other or same plant lines or species, or by artificially programming mutations of nucleotide sequences coding for native citrus polypeptides. Alterations of the native amino acid sequence can be accomplished by any of a number of conventional methods. Mutations can be introduced at particular loci by synthesizing oligonucleotides containing a mutant sequence, flanked by restriction sites enabling ligation to fragments of the native sequence. Following ligation, the resulting reconstructed sequence encodes an analog having the desired amino acid insertion, substitution, or deletion. Alternatively, oligonucleotide-directed site-specific mutagenesis procedures can be employed to provide an altered gene wherein predetermined codons can be altered by substitution, deletion or insertion.

In one embodiment, the invention contemplates: vectors comprising the nucleic acids of the invention. For example, a vector comprising at least one nucleic acid chosen from (a) a nucleic acid comprising the nucleotide sequence substantially as set out in SEQ ID NO:6, SEQ ID NO:7, SEQ ID NO:8, SEQ ID NO:9, or SEQ ID NO:10; (b) a nucleic acid encoding the polypeptide substantially set out in SEQ ID NO:1, SEQ ID NO:2, SEQ ID NO:3, SEQ ID NO:4, or SEQ ID NO:5; and (c) a nucleic acid that hybridizes to the nucleic acid of (a) or (b) under low stringency conditions, wherein the polypetide encoded by said nucleic acid is a sesquiterpene synthase.

A vector as used herein includes any recombinant vector including but not limited to viral vectors, bacteriophages and plasmids.

Recombinant expression vectors containing a nucleic acid sequence of the invention can be prepared using well known methods. In one embodiment, the expression vectors include a cDNA sequence encoding the polypeptide operably linked to suitable transcriptional or translational regulatory nucleotide sequences, such as those derived from a mammalian, microbial, viral, or insect gene. Examples of regulatory sequences include transcriptional promoters, operators, or enhancers, mRNA ribosomal binding sites, and appropriate sequences which control transcription and translation initiation and termination. Nucleotide sequences are "operably linked" when the regulatory sequence functionally relates to the cDNA sequence of the invention. Thus, a promoter nucleotide sequence is operably linked to a cDNA sequence if the promoter nucleotide sequence controls the transcription of the cDNA sequence. The ability to replicate in the desired host cells, usually conferred by an origin of replication, and a selection gene by which transformants are identified can additionally be incorporated into the expression vector.

In addition, sequences encoding appropriate signal peptides that are not naturally associated with the polypeptides of the invention can be incorporated into expression vectors. For example, a DNA sequence for a signal peptide (secretory leader) can be fused in-frame to a nucleotide sequence of the invention so that the polypeptides of the invention is initially translated as a fusion protein comprising the signal peptide. A signal peptide that is functional in the intended host cells enhances extracellular secretion of the expressed polypeptide. The signal peptide can be cleaved from the polypeptide upon secretion from the cell.

Fusions of additional peptide sequences at the amino and carboxyl terminal ends of the polypeptides of the invention can be used to enhance expression of the polypeptides or aid in the purification of the protein.

In one embodiment, the invention includes a host cell comprising a nucleic acid of the invention. Another embodiment of the invention is a method of making a recombinant host cell comprising introducing the vectors of the invention, into a host cell. In a further embodiment, a method of producing a polypeptide comprising culturing the host cells of the invention under conditions to produce the polypeptide is contemplated. In one embodiment the polypeptide is recovered. The methods of invention include methods of making at least one sesquiterpene synthase of the invention comprising culturing a host cell comprising a nucleic acid of the invention, and recovering the sesquiterpene synthase accumulated.

Suitable host cells for expression of polypeptides of the invention include prokaryotes, yeast or higher eukaryotic cells. Appropriate cloning and expression vectors for use with bacterial, fungal, yeast, and mammalian cellular hosts are described, for example, in Pouwels et al., Cloning Vectors: A Laboratory Manual, Elsevier, New York, (1985). Cell-free translation systems could also be employed to produce the disclosed polypeptides using RNAs derived from DNA constructs disclosed herein.

Prokaryotes include gram negative or gram positive organisms, for example, E. coli or Bacilli. Suitable prokaryotic host cells for transformation include, for example, E. coli, Bacillus subtilis, Salmonella typhimurium, and various other species within the genera Pseudomonas, Streptomyces, and Staphylococcus. In a prokaryotic host cell, such as E. coli, the polypeptides can include a N-terminal methionine residue to facilitate expression of the recombinant polypeptide in the prokaryotic host cell. The N-terminal methionine can be cleaved from the expressed recombinant polypeptide.

Examples of useful expression vectors for prokaryotic host cells include those derived from commercially available plasmids such as the cloning vector pET plasmids (Novagen, Madison, WI, USA) or yet pBR322 (ATCC 37017). pBR322 contains genes for ampicillin and tetracycline resistance and thus provides simple means for identifying transformed cells. To construct an expression vector using pBR322, an appropriate promoter and a DNA sequence encoding one or more of the polypeptides of the invention are inserted into the pBR322 vector. Other commercially available vectors include, for example, pKK223-3 (Pharmacia Fine Chemicals, Uppsala, Sweden) and pGEM-1 (Promega Biotec, Madison, WI, USA). Other commercially available vectors include those that are specifically designed for the expression of proteins; these would include pMAL-p2 and pMAL-c2 vectors that are used for the expression of proteins fused to maltose binding protein (New England Biolabs, Beverly, MA, USA).

Promoter sequences commonly used for recombinant prokaryotic host cell expression vectors include bacteriophage T7 promoter (Studier F.W. and Moffatt B.A., J. Mol. Biol. 189:113, 1986), β-lactamase (penicillinase), lactose promoter system (Chang et al., Nature 275:615, 1978; and Goeddel et al., Nature 281:544, 1979), tryptophan (trp) promoter system (Goeddel et al., Nucl. Acids Res. 8:4057, 1980; and EP-A-36776), and tac promoter (Maniatis, MoLecular Cloning: A Laboratory Manual, Cold Spring Harbor Laboratory, p. 412, 1982). A particularly useful prokaryotic host cell expression system employs a phage λ PL promoter and a cl857ts thermolabile repressor sequence. Plasmid vectors available from the American Type Culture Collection ("ATCC"), which incorporate derivatives of the PL promoter, include plasmid pHUB2 (resident in E. coli strain JMB9 (ATCC 37092)) and pPLc28 (resident in E. coli RR1 (ATCC 53082)).

Polypeptides of the invention can also be expressed in yeast host cells, preferably from the Saccharomyces genus (e.g., S. cerevisiae). Other genera of yeast, such as Pichia or Kluyveromyces (e.g. K. lactis ), can also be employed. Yeast vectors will often contain an origin of replication sequence from a 2µ yeast plasmid, an autonomously replicating sequence (ARS), a promoter region, sequences for polyadenylation, sequences for transcription termination, and a selectable marker gene. Suitable promoter sequences for yeast vectors include, among others, promoters for metallothionine, 3-phosphoglycerate kinase (Hitzeman et al., J. Biol. Chem. 255:2073, 1980), or other glycolytic enzymes (Hess et al., J. Adv. Enzyme Reg. 7:149, 1968; and Holland et al., Biochem. 17:4900, 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other suitable vectors and promoters for use in yeast expression are further described in Hitzeman, EPA-73,657 or in Fleer et. al., Gene, 107:285-195 (1991); and van den Berg et. al., Bio/Technology, 8:135-139 (1990). Another alternative is the glucose-repressible ADH2 promoter described by Russell et al. (J. Biol. Chem. 258:2674, 1982) and Beier et al. (Nature 300:724, 1982). Shuttle vectors replicable in both yeast and E. coli can be constructed by inserting DNA sequences from pBR322 for selection and replication in E. coli (Ampr gene and origin of replication) into the above-described yeast vectors.

One embodiment of the invention is a non-human organism modified to harbor a nucleic acid of the invention. The non-human organism and/or host cell may be modified by any methods known in the art for gene transfer including, for example, the use of deliver devices such as lipids and viral vectors, naked DNA, electroporation and particle-mediated gene transfer. In one embodiment, the non-human organism is a plant, insect or microorganism.

For example, in one embodiment the invention provides a method of making at least one sesquiterpene synthase comprising culturing a host modified to contain at least one nucleic acid under conditions conducive to the production of said at least one sesquiterpene synthase wherein said at least one nucleic acid is chosen from (a) a nucleic acid comprising the nucleotide sequence substantially as set out in SEQ ID NO:6, SEQ ID NO:7, SEQ ID NO:8, SEQ ID NO:9, or SEQ ID NO:10; (b) a nucleic acid encoding the polypeptide substantially set out in SEQ ID NO:1, SEQ ID NO:2, SEQ ID NO:3, SEQ ID NO:4, or SEQ ID NO:5; and (c) a nucleic acid that hybridizes to the nucleic acid of (a) or (b) under low stringency conditions, wherein the polypetide encoded by said nucleic acid is a sesquiterpene synthase.

In a further embodiment, the host is a plant such as tobacco, animal or microorganism also including but not limited to bacterial cells, yeast cells, plant cells, and animal cells. As used herein, plant cells and animals cells include the use of plants and animals as a host. For example, in some embodiments of the invention, expression is in a genetically modified non-human organism.

In one embodiment, mammalian or insect host cell culture systems are employed to express recombinant polypeptides of the invention. Baculovirus systems for production of heterologous proteins in insect cells are reviewed by Luckow and Summers, Bio/Technology 6:47 (1988). Established cell lines of mammalian origin also can be employed. Examples of suitable mammalian host cell lines include the COS-7 line of monkey kidney cells (ATCC CRL 1651) (Gluzman et al., Cell 23:175, 1981), L cells, C127 cells, 3T3 cells (ATCC CCL 163), Chinese hamster ovary (CHO) cells, HeLa cells, and BHK (ATCC CRL 10) cell lines, and the CV-1/EBNA-1 cell line (ATCC CRL 10478) derived from the African green monkey kidney cell line CVI (ATCC CCL 70) as described by McMahan et al. (EMBO J. 10: 2821 ,1991).

Established methods for introducing DNA into mammalian cells have been described (Kaufman, R.J., Large Scale Mammalian Cell Culture, 1990, pp. 15-69). Additional protocols using commercially available reagents, such as Lipofectamine (Gibco/BRL) or Lipofectamine-Plus, can be used to transfect cells (Felgner et al., Proc. Natl. Acad. Sci. USA 84:7413-7417, 1987). In addition, electroporation can be used to transfect mammalian cells using conventional procedures, such as those in Sambrook et al. Molecular Cloning: A Laboratory Manual, 2 ed. Vol. 1-3, Cold Spring Harbor Laboratory Press, 1989). Selection of stable transformants can be performed using resistance to cytotoxic drugs as a selection method. Kaufman et al., Meth. in Enzymology 185:487-511 , 1990, describes several selection schemes, such as dihydrofolate reductase (DHFR) resistance. A suitable host strain for DHFR selection can be CHO strain DX-B11 , which is deficient in DHFR (Urlaub and Chasin, Proc. Natl. Acad. Sci. USA 77:4216-4220, 1980). A plasmid expressing the DHFR cDNA can be introduced into strain DX-B11, and only cells that contain the plasmid can grow in the appropriate selective media.

Transcriptional and translational control sequences for mammalian host cell expression vectors can be excised from viral genomes. Commonly used promoter sequences and enhancer sequences are derived from polyoma virus, adenovirus 2, simian virus 40 (SV40), and human cytomegalovirus. DNA sequences derived from the SV40 viral genome, for example, SV40 origin, early and later promoter, enhancer, splice, and polyadenylation sites can be used to provide other genetic elements for expression of a structural gene sequence in a mammalian host cell. Viral early and late promoters are particularly useful because both are easily obtained from a viral genome as a fragment, which can also contain a viral origin of replication (Fiers et al., Nature 273:113, 1978; Kaufman, Meth. in Enzymology, 1990).

There are several methods known in the art for the creation of transgenic plants. These include, but are not limited to: electroporation of plant protoplasts, liposome-mediated transformation, polyethylene-glycol-mediated transformation, microinjection of plant cells, and transformation using viruses. In one embodiment, direct gene transfer by particle bombardment is utilized.

Direct gene transfer by particle bombardment provides an example for transforming plant tissue. In this technique a particle, or microprojectile, coated with DNA is shot through the physical barriers of the cell. Particle bombardment can be used to introduce DNA into any target tissue that is penetrable by DNA coated particles, but for stable transformation, it is imperative that regenerable cells be used. Typically, the particles are made of gold or tungsten. The particles are coated with DNA using either CaCl2 or ethanol precipitation methods which are commonly known in the art.

DNA coated particles are shot out of a particle gun. A suitable particle gun can be purchased from Bio-Rad Laboratories (Hercules, CA). Particle penetration is controlled by varying parameters such as the intensity of the explosive burst, the size of the particles, or the distance particles must travel to reach the target tissue.

The DNA used for coating the particles may comprise an expression cassette suitable for driving the expression of the gene of interest that will comprise a promoter operably linked to the gene of interest.

Methods for performing direct gene transfer by particle bombardment are disclosed in U.S. Patent 5,990,387 to Tomes et al.

In one embodiment, the cDNAs of the invention may be expressed in such a way as to produce either sense or antisense RNA. Antisense RNA is RNA that has a sequence which is the reverse complement of the mRNA (sense RNA) encoded by a gene. A vector that will drive the expression of antisense RNA is one in which the cDNA is placed in "reverse orientation" with respect to the promoter such that the non-coding strand (rather than the coding strand) is transcribed. The expression of antisense RNA can be used to down-modulate the expression of the protein encoded by the mRNA to which the antisense RNA is complementary. Vectors producing antisense RNA's could be used to make transgenic plants, as described above.

In one embodiment, transfected DNA is integrated into a chromosome of a non-human organism such that a stable recombinant systems results. Any chromosomal integration method known in the art may be used in the practice of the invention, including but not limited to, recombinase-mediated cassette exchange (RMCE), viral site specific chromosomal insertion, adenovirus, and pronuclear injection.

A further embodiment of the invention is methods of making terpenoids and sesquiterpene compounds, for example, using the nucleotides and polypeptides of the invention. Examples include methods of making at least one terpenoid comprising contacting at least one acyclic pyrophosphate terpene precursor with at least one polypeptide encoded by a nucleic acid chosen from (a) a nucleic acid comprising the nucleotide sequence substantially as set out in SEQ ID NO:6, SEQ ID NO:7, SEQ ID NO:8, SEQ ID NO:9, or SEQ ID NO:10; (b) a nucleic acid encoding the polypeptide substantially set out in SEQ ID NO:1, SEQ ID NO:2, SEQ ID NO:3, SEQ ID NO:4, or SEQ ID NO:5; and (c) a nucleic acid that hybridizes to the nucleic acid of (a) or (b) under low stringency conditions, wherein the polypetide encoded by said nucleic acid is a sesquiterpene synthase, and isolating at least one terpenoid produced. Another example is a method of making at least one terpenoid comprising contacting at least one acyclic pyrophosphate terpene precursor with at least one polypeptide substantially set out in SEQ ID NO:1, SEQ ID NO:2, SEQ ID NO:3, SEQ ID NO:4, or SEQ ID NO:5 and isolating at least one terpenoid produced.

As used herein an acyclic pyrophosphate terpene precursor is any acyclic pryrophosphate compound that is a precursor to the production of at least one terpene including but not limited to geranyl-pyrophosphate (GPP), farnesyl-pyrophosphate (FPP) and geranylgeranyl-pyrophosphate (GGPP).

In one embodiment, the at least one terpenoid is chosen from sesquiterpenes. In one embodiment, the at least one acyclic pyrophosphate terpene precursor is farnesyl-pyrophosphate. In a further embodiment, the at least one sesquiterpenes is chosen from bicylogermacrene((E,E)-3,7,11,11-tetramethyl-bicyclo[8.1.0]undeca-2,6-diene), cubebol ((1R,4S,5R,6R,7S,10R)-7-isopropyl-4, 10-dimethyl-tricyclo[4.4.0.0(1,5)]decan-4-ol, valencene ((+)-(1R)-1,2,3,5,6,7,8,8A-octahydro-7-isopropenyl-1 @,8A@-dimethylnaphthaalene), α-cubebene, germacrene D, and δ - cadinene(rel-(1R,8AS)-1,2,3,5,6,8A-hexahydro-1-isopropyl-4,7-dimethylnaphtalene) (Figure 2). The terpenoids of the invention may be isolated by any method used in the art including but not limited to chromatography, extraction and distillation.

In one embodiment, the distribution of products or the actual products formed may be altered by varying the pH at which the synthase contacts the acyclic pyrophosphate terpene precursor, such as, for example, farnesyl-pyrophosphate. In one embodiment, the pH is 7. In a further embodiment the pH is less than 7, such as, for example, 6, 5, 4, and 3.

Also within the practice of the invention is an organism (e.g.,. micro-organism or plant) that is used to construct a platform for high level production of a substrate of sesquiterpene synthases (e.g., FPP) and the introduction of a nucleic acid of the invention into the organism. For example, at least one nucleic acid of the invention that encodes a sesquiterpene synthase is incorporated into a non-human organism that produces FPP thereby effecting conversion of FPP to a sesquiterpene, and the subsequent metabolic production of the sesquiterpene. In one embodiment, this results in a platform for the high level production of sesquiterpenes.

In one embodiment, the nucleic acids of the invention are used to create other nucleic acids coding for sesquiterpene synthases. For example, the invention provides for a method of identifying a sesquiterpene synthases comprising constructing a DNA library using the nucleic acids of the invention, screening the library for nucleic acids which encode for at least one sesquiterpene synthase. The DNA library using the nucleic acids of the invention may be constructed by any process known in the art where DNA sequences are created using the nucleic acids of the invention as a starting point, including but not limited to DNA suffling. In such a method, the library may be screened for sesquiterpene synthases using a functional assay to find a target nucleic acid that encodes a sesquiterpene synthase. The activity of a sesquiterpene synthase may be analyzed using, for example, the methods described herein. In one embodiment, high through put screening is utilized to analyze the activity of the encoded polypeptides.

As used herein a "nucleotide probe" is defined as an oligonucleotide or polynucleotide capable of binding to a target nucleic acid of complementary sequence through one or more types of chemical bonds, through complementary base pairing, or through hydrogen bond formation. As described above, the oligonucleotide probe may include natural (ie. A, G, C, or T) or modified bases (7-deazaguanosine, inosine, etc.). In addition, bases in a nucleotide probe may be joined by a linkage other than a phosphodiester bond, so long as it does not prevent hybridization. Thus, oligonucleotide probes may have constituent bases joined by peptide bonds rather than phosphodiester linkages.

A "target nucleic acid" herein refers to a nucleic acid to which the nucleotide probe or molecule can specifically hybridize. The probe is designed to determine the presence or absence of the target nucleic acid, and the amount of target nucleic acid. The target nucleic acid has a sequence that is complementary to the nucleic acid sequence of the corresponding probe directed to the target. As recognized by one of skill in the art, the probe may also contain additional nucleic acids or other moieties, such as labels, which may not specifically hybridize to the target. The term target nucleic acid may refer to the specific nucleotide sequence of a larger nucleic acid to which the probe is directed or to the overall sequence (e.g., gene or mRNA). One skilled in the art will recognize the full utility under various conditions.

Other than in the operating example, or where otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the specification and claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. The following examples are intended to illustrate the invention without limiting the scope as a result. The percentages are given on a weight basis.

The following examples are intended to illustrate the invention without limiting the scope as a result.

### Examples

### Material

A grapefruit (Citrus paradisi) flavedo was used as starting material for the experiments described herein. The flavedo (external, colored portion of the fruit peel) contains the oil glands that are the site of terpene biosynthesis. The grapefruit flavedo was prepared at Simone Gatto (Sicily) from freshly picked ripening fruits. The flavedo (3-4 mm thickness) was cutoff, immediately frozen and kept frozen during the transport and all subsequent steps in order to prevent from degradation.

### Example 1 : Isolating Sesquiterpene Synthase cDNA using RT-PCR

The deduced amino-acid sequences of plant sesquiterpene synthases were aligned to identify conserved regions and design plant sesquiterpene synthases-specific oligonucleotides. In order to obtain better sequence homology, the sequences were separated into two groups (Figure 4). The first group contained the sequences of the Germacrene C synthase from Lycopersicon esculentum cv. VFNT cherry (Colby et al, 1998), the (E)-β-farnesene synthase from Mentha x piperita (Crock et al, 1997), the δ-selinene synthase from Abies grandis (Steele et al, 1998), a sesquiterpene synthase from Citrus junos (GenBank accession no. AF288465) the 5-epi aristolochene synthases from Nicotiana tabacum (Facchini and Chappell, 1992) and from Capsicum annuum (Back et al, 1998), the vetispiradiene synthases from Solanum tuberosum and from Hyoscyamus muticus (Back and Chappel, 1995). The second group contained sequences of the (+)-δ-cadinene synthases from Gossypium arboreum (Chen et al. 1995), the amorpha-4,11-diene synthase (Mercke et al, 2000) and the epi-cedrol synthase (Merck et al, 1999) from Artemisia annua and the γ-humulene synthase from Abies grandis (Steele et al, 1998). The highest sequence homology was found in the central part of the sequences. Three regions containing sufficiently conserved amino-acids were selected and degenerated oligonucleotides specific for these regions were designed (i.e. two forward and one reverse primers were deduced from each alignment) (Figure 4).

RT-PCR was performed using total RNA from grapefruit flavedo prepared by the Hot Borate technique and the different combination of the forward and reverse degenerated primers. The Hot Borate method for total RNA extraction was adapted from Wan and Wilkins (Wan et al. (1994) Anal. Biochem. 223, 7-12.). Tissues were crushed to a fine powder in liquid nitrogen using a mortar and pestle. The powder was added to 5 ml extraction buffer (200 mM borate, 30 mM EGTA, 10 mM DTT, 1% SDS, 1% NA deoxycholate, 2% PVP, 0.5% nonidet NP-40) preheated at 80°C. The mixture was homogenized using an UltraturaxTM homogenizer and filtered through two layer of MiraclothTM filter (Calbiochem). The mixture was incubated 90 minutes at 42°C in the presence of 0.5 mg/ml proteinase K (for protein/ribonuclease digestion). KCI was then added to 1 M final concentration and, after 1 hour incubation on ice, the mixture was placed in a centrifuge for 10 min at 10000g and 4°C. The supernatant was recovered and 1/3 volume of 8M LiCl was added. The mixture was incubated over night at 4°C and placed in a centrifuge for 20 minutes at 10000g and 4°C. The supernatant was discarded and, the pellet washed with 2M LiCl and centrifuged again as before. The pellet was then resuspended in RNase-free distillated water, and 0.15 vol of 2 M potassium acetate solution and 2.5 volumes absolute ethanol were added. The RNA were precipitated by incubating 2 hours at -20°C and pelleted by centrifugation as before. The RNA pellet was washed with 80% ethanol and resuspended in RNase free distillated water.

The concentration of RNA was estimated from the OD at 260 nm. The integrity of the RNA was evaluated on an agarose gel by verifying the integrity of the ribosomic RNA bands.

RT-PCR was performed using the Qiagen OneStep RT-PCR Kit and an Eppendorf Mastercycler gradiant thermal cycler. Typical reaction mixtures contained 10 µl 5X Qiagen OneStep RT-PCR buffer, 400 µM each dNTP, 400 nM each primer, 2 µl Qiagen OneStep RT-PCR Enzyme Mix, 1 µl RNasin® Ribonuclease Inhibitor (Promega Co.) and 1 µg total RNA in a final volume of 50 µl. The thermal cycler conditions were: 30 min at 50°C (reverse transcription); 15 min at 95°C (DNA polymerase activation); 40 cycles of 45 sec at 94°C, 10 sec at 42°C to 45°C (depending on the primer used), 45 sec to 90 sec (depending on the size of the DNA fragment to be amplified) at 72°C; and 10 min at 72°C.

The size of the PCR products was evaluated on a 1 % agarose gel. The bands corresponding to the expected size were excised from the gel, purified using the QIAquick® Gel Extraction Kit (Qiagen) and cloned in the pCR®2.1-TOPO vector using the TOPO TA cloning Kit (Invitrogen). Inserted cDNAs were then subject to DNA sequencing and the sequence compared against the GenBank non redundant protein database (NCBI) using the BLASTX algorithm (Altschul et al 1990).

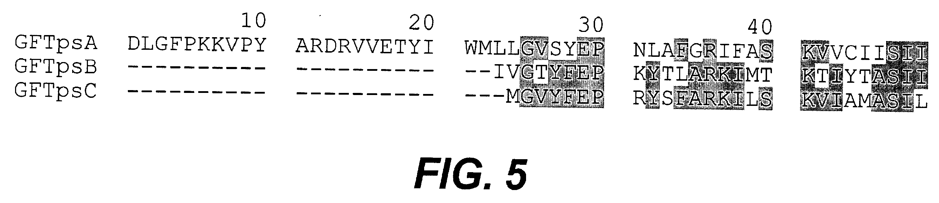

The analysis by DNA sequencing and the Blast search of the PCR products with expected length (80 to 240 bp in size) revealed fragments of three different sesquiterpene synthases, which were named GFTpsA, GFtpsB and GFTpsC. The 180-bp GFTpsA fragment was amplified with the primers TpsVFI and TpsVR3, the 115-bp GFtpsB fragment was amplified with the primers TpsVF2 and TpsVR3, and the 115-bp GFTpsA fragment was amplified with the primers TpsVFI and TpsVR3. The deduced amino-acid sequences revealed significant differences between these three fragments (Figure 5).

### Example 2: Isolating Sesquiterpene Synthase cDNA using 5'/3'-RACE

To isolate the full- length sequences of the sesquiterpene synthases, a 5'/3'-RACE approach was first used. cDNA was synthesized using Marathon™ cDNA Amplification Kit (Clontech) and starting from 1 µg mRNA purified from total RNA prepared with the Hot Borate technique. The quality of the synthesized cDNA was poor, essentially small size cDNAs were obtained (average size of 0.5 Kb). However, using this cDNA, the 3'-end of the GFTpsB and GFTpsC were obtained using the gene specific primers GFTpsBRFI and GFTpsBRF2 for GFTpsB and the gene specific primers GFTpsCRFI and GFTpsCRF2 for GFTpsC (see Table 1). mRNA prepared by the guanidinium-thiocyanate/phenol extaction method gave higher quality cDNA with an average size of 2 Kb and allowed the isolation of the 5'-end sequence of GFTpsC using the gene specific primers GFTpsCRRI and GFTpsRR2 (see Table 1), completing the full- length sequence of this clone. The 5'-end of GFTpsB and the 5'-end and 3'-end of GFTpsA could not be obtained by this approach.

The guanidinium-thiocyanate/phenol extraction method was based on the technique described by Chomczynski and Sacchi using the RNAClean™ solution from ThermoHybaid (Chomczynski, P., and Sacchi, N., (1987) Anal. Biochem. 162, 156-159.). Briefly, 2 g of frozen tissues was crushed to a fine powder in liquid nitrogen using a mortar and pestle. The powder was transferred to 20 ml RNAClean™ solution and the suspension was homogenized using an UltraturaxTM homogenizer and filtered through MiraclothTM filter (Calbiochem). After addition of 0.1 volume of chloroform, the tube was placed on ice and centrifuged 20 min at 12000g and 4°C. The upper aqueous phase was recovered and one volume of isopropanol was added. After 20 min. incubation at -20°C, the sample was centrifuged 20 min. at 12000g and 4°C. The large white pellet obtained was washed with 70% ethanol and dried at room temperature. This pellet, containing the total RNA, was immediately subject to mRNA purification by oligodT-cellulose affinity chromatography using the FastTrack® 2.0 mRNA isolation Kit (Invitrogen). The manufacturer's protocol was followed except that after resuspension of the total RNA in the lysis buffer, the sample was heated at 100°C instead of 65°C.

3' and 5' rapid amplification of cDNA ends (RACE) were performed using Marathon™ cDNA Amplification Kit (Clontech). The procedure began with the first-strand cDNA synthesis starting from mRNA using an oligo(dT) primer. After second-strand synthesis, specific adaptors were ligated to the double stranded cDNA (ds cDNA) ends. This procedure provided an uncloned library of adaptor-ligated ds cDNA. Purified grapefruit mRNA (1 µg) was used as starting material. The quality and quantity of cDNA was evaluated on an agarose gel.

The 3'- or 5'-end of the specific cDNAs were amplified with Advantage® 2 Polymerase Mix using a combination of gene- and adaptor-specific oligonucleotides. Typical RACE reaction mixtures contained, in a final volume of 50 µl, 5 µl 10X cDNA PCR Reaction Buffer (clontech), 200 nM each dNTP, 1 µl Advantage® 2 Polymerase Mix, 200 µM adaptor-specific primer (Clontech), 200 µM gene-specific primer (see Table 1) and 5 µl of 50 to 250 fold diluted adaptor-ligated cDNA. Amplification was performed on an Eppendorf Mastercycler gradiant thermal cycler. The thermal Cycling conditions were as follows: 1 min at 94°C, 5 cycles of 30 sec at 94°C and 2 to 4 min at 72°C, 5 cycles of 30 sec at 94°C and 2 to 4 min at 70°C, 20 cycles of 30 sec at 94°C and 2 to 4 min at 68°C. When necessary a second round of amplification was performed using a nested adaptor-specific primer (Clontech) and a nested gene-specific primer.

The amplification products were evaluated, sub-cloned, and the sequence analyzed as above for the RT-PCR products.

**TABLE 1: The following primers were used in the 3'/5'-RACE experiments:**

| Name. | Description. | Sequence (5' to 3'). |
|---|---|---|
| GFTpsARF1 (SEQ ID NO:33) | 3'-RACE forward primer. | CTGGGAGTGTCCTATGAGCT CAATTTGG |
| GFTpsARF2 (SEQ ID NO:34) | GFTpsA 3'-RACE forward nested primer. | GTAGAATTTTTGCATCCAAAG TGGTGTGC |
| GFTpsARR1 (SEQ ID NO:35) | GFTpsA 5'-RACE reverse primer. | CACACCACTTTGGATGCAAA AATTCATC |
| GFTpsARR2 (SEQ ID NO:36) | GFTpsA 5'-RACE reverse nested primer. | CCAAATTGGGCTCATAGGAC ACTCCCAG |
| GFTpsBRF1 (SEQ ID NO:37) | GFTpsB 3'-RACE forward primer. | TAGGGACGTATTTTGAACCA AAGTAC |
| GFTpsBRF2 (SEQ ID NO:38) | GFTpsB 3'-RACE forward nested primer. | AAATAATGACCAAAACAATTT ACACGG |
| GFTpsBRR1 | GFTpsB 5'-RACE reverse primer. | GCACTTTCATGTATTCTGGAA |
| (SEQ ID NO:39) | | G |
| GFTpsBRR2 (SEQ ID NO:40) | GFTpsB 5'-RACE reverse nested primer. | GTTTGAGCTCTTCAAAGAAA CC |
| GFTpsCRF1 (SEQ ID NO:41) | GFTpsC 3'-RACE forward primer. | AATGGGAGTGTATTTTGAGC CTCGATACTCC |
| GFTpsCRF2 (SEQ ID NO:42) | GFTpsC 3'-RACE forward nested primer. | GATATTTTCCAAAGTAATTGC AATGGCATCC |
| GFTpsCRR1 (SEQ ID NO:43) | GFTpsC 5'-RACE reverse primer. | GTTGCTAATATCCCACCTTTT GATAGC |
| GFTpsCRR2 (SEQ ID NO:44) | GFTpsC 5'-RACE reverse nested primer. | AAGTGTGCCATAGGCGTCGT AGG |
| GFTpsDRR1 (SEQ ID NO:45) | GFTpsD 5'-RACE reverse primer. | CTGTTCCGCAAGCTTAGGGG TTACATG |
| GFTpsDRR2 (SEQ ID NO:46) | GFTpsD 5'-RACE reverse nested primer. | CTGAGCTACCAATGACTTCA GGTGAGTGG |
| GFTpsERR1 (SEQ ID NO:47) | GFTpsE 5'-RACE reverse primer. | CAATTTTTGCCATACACATCAT AGATATCATC |
| GFTpsERR2 (SEQ ID NO:48) | GFTpsE 5'-RACE reverse nested primer. | AACAGAAGTCATGGAGATCA CTTTCGTC |
| GFTpsERR3 (SEQ ID NO:49) | GFTpsE 5'-RACE reverse primer. | CGCAAGAGATGTTTTAAAGTT CCCATCC |
| GFTpsERR4 (SEQ ID NO:50) | GFTpsE 5'-RACE reverse nested primer. | TGAACATCAGCGGAAATTTTA TAGCC |

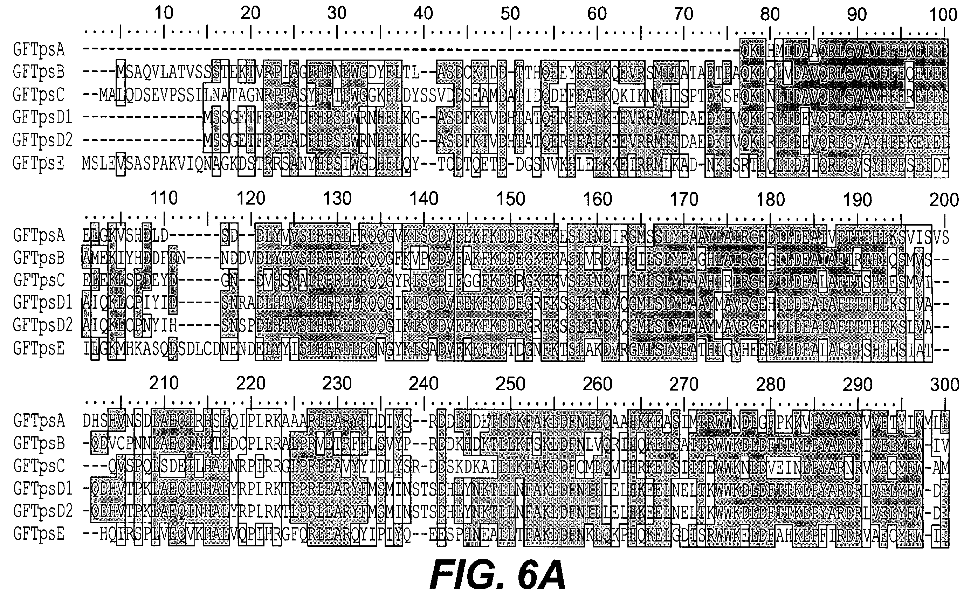

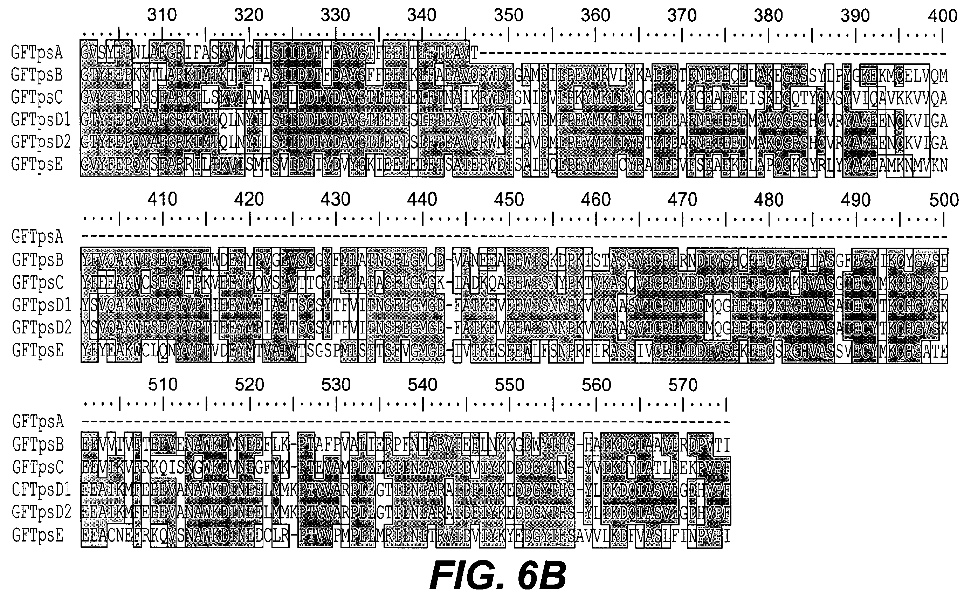

The partial GFTpsB cDNA obtained by 3'-RACE revealed high sequence identity to a putative terpene synthase cDNA found in the public databases (GeneBank accession no. AF288465) and isolated from Citrus junos. Primers specific for the sequence of this terpene synthase were designed: one pair of forward and reverse primers (junosFI and junosRI) designed against the non-coding region of the C. junos terpene synthase and one pair of forward and reverse primers (junosF2 and junos R2) designed in the coding region including the start and stop codons. PCR on the grapefruit Marathon™ cDNA library using the primers junosFI and junosRI produced no amplicon. Using the junosF2 and junosR2 primers a fragment of 1.6 Kb was amplified. DNA sequencing confirmed this PCR product as being a 1669-bp full-length sesquiterpene synthase of the GFTpsB clone. (Figure 6).

### Example 3: cDNA library screening and EST sequencing

In order to obtain the full-length cDNA of the partial clones obtained by the PCR approach, a cDNA library prepared from grapefruit flavedo mRNA was constructed.

cDNA synthesis and library construction were performed using the Uni-ZAP® XR library construction Kit (Stratagene) according to manufacturer's protocol starting from 7.5 µg grapefruit flavedo mRNA (prapared by the Guanidinium-Thiocyanate/Phenol method). The original titer of the library was 2x10⁷ PFU (plaques forming units) and the average insert size was 1.1 Kb.

Two approaches were used to isolate sesquiterpene synthases encoding cDNAs from the cDNA library : EST sequencing and screening using a DNA probe. For EST (expressed sequence tag) sequencing, a fraction of the library was used to excise the pBluescript phagemid from the Uni-ZAP XR vector according to Stratagene's mass excision protocol. Resulting transformed bacterial colonies (576) were randomly picked and the plasmid purified. One sequencing reaction was performed for each clone using the T3 primer. The sequences were first edited to remove vector sequences and compared against the GenBank non redundant protein database (NCBI) using the BLASTX algorithm (Altschul et al 1990).

The library was screened with digoxigenin (DIG)-labeled DNA probes. The probes were synthesized by incorporation of DIG-dUTP into a DNA fragment by PCR using the PCR DIG (digoxigenin) Probe Synthesis Kit (Roche Diagnostics). A 149-bp GFTpsA-derived DNA probe was amplified from an aliquot of the library using the forward primer GFTpsAproF (SEQ ID NO:51) (5'-ACGATTTAGGCTTCCCTAAAAAGG-3') and the reverse primer GFTpsAproR (SEQ ID NO:52) (5'-TATTATGGAATATTATGCACACCAC-3'). A 1036-bp GFTpsB-derived probe was amplified from the pET-GFTpsB2-2 plasmid using the forward primer junosF2 (SEQ ID NO:53) (5'-AAATGTCCGCTCAAGTTCTAGCAACGG-3') and the reverse primer GFTpsBRR2 (SEQ ID NO:54) (5'-GTTTGAGCTCTTCAAAGAAACC-3'). A 1008-bp GFTpsC-derived DNA probe was amplified from the pET-GFTpsC plasmid using the forward primer GFTpsCpetFI (SEQ ID NO:55) (5'-ATGGCACTTCAAGATTCAGAAGTTCC-3') and the reverse primer GFTpsCRRI (SEQ ID NO:56) (5'-GTTGCTAATATCCCACCTTTTGATAGC-3').

The probes were hybridized to plaques lifts, prepared from plates containing 5,000 to 25,000 PFU, at 40°C in Dig Easy Hyb hybridization solution (Roche Diagnostics). The detection of the probe-target hybrids on the membranes was performed by chemiluminescence using Anti-Digoxigenin-Alkaline Phosphatase (Roche Diagnostics) and CDP-Star alkaline phosphate substrate (Roche Diagnostics), and the visualization was made on a VersaDoc Imaging System (BioRad).

Phages from positives signals were cored from the agar plates and subject to secondary and if necessary to tertiary screening to achieve single plaques positive signals. The positive isolates were in vivo excised and the insert sequenced as described above

The library was screened using DNA probes prepared from GFTpsA, GFTpsB and GFTpsC. This approach yielded three different sesquiterpene synthase cDNAs. Two of them were previously found clones: one clone, named GF2-30-1 , was a 5'-end 300-bp truncated form of the GFTpsC cDNA; the second clone, named 9-13-6, was a partial clone of GFtpsA that was truncated at its 5'-end by approximately 168-bp as judged by comparison to other sesquiterpene synthases. The 9-13-6 clone provided 618-bp additional 5'-end sequence information compared to the above mentioned partial GFTpsA clone obtained by RT-PCR. At its 3'-end, the 9-13-6 clone was also incomplete. Approximately 680 nucleotides were missing and were replaced by a 600-bp fragment of no defined function. The third sesquiterpene synthase encoding cDNA obtained by screening, GF2-5-11, encoded a new sesquiterpene synthase that was named GFTpsD. This clone was truncated at the 5'-end, but the missing 414-bp was recovered by 5'-RACE using the primers described in Table 1. The full-length GFTpsD was then amplified from Marathon™ cDNA library and cloned in the bacterial expression vector as described above. Analysis of the DNA sequence of several full-length GFTpsD cDNAs revealed that two closely related sesquiterpene synthases with minor sequence differences were present, they were named GFTpsDI and GFTpsD2 (Figure 6). The deduced amino-acid sequence of these two variants differed from each other by 4 residues.

For the EST sequencing approach, 576 clones were sequenced. Among them, only three clones encoded putative terpene-synthases like proteins and more precisely, two different sesquiterpene synthase-like and one monoterpene synthase-like proteins. One of the two sesquiterpene synthase-like clones (clone GF002-G3) was the previously identified clone GFTpsDI. The second (clone GF006-G7) was a truncated cDNA encoding a new sesquiterpene synthase that was named GFTpsE. This clone was again 5'-end truncated (by approximately 800 bp) and the missing fragment could be amplified by 5'-RACE in two stages as follows. A first 5'-RACE using the primers GFTpsERRI and GFTpsERR2 (see Table 1) provided 500 additional 5'-end nucleotides and a second 5'RACE using the primers GFTpsERR3 and GFTpsERR4 (see Table 1) provided the missing 5'-end DNA sequence to reconstitute the full-length GFTpsE cDNA (Figure 6).

### Example 4: Construction of Plasmids and Enzyme expression

### Construction of expression plasmids.

The cDNA were sub-cloned in the pET11 a expression plasmid (Novagen) for functional expression of the sesquiterpene synthases. For GFTpsB, GFtpsD and GFTpsE, the full-length cDNAs were amplified by PCR to introduce an Ndel site at the 5'-end including the start codon and a BamHI site at the 3'-end immediately after the stop codon. For GFTpsB, the forward primer GFTpsBNdel (SEQ ID NO:57) 5'-GCATGTTCCATATGTCCGCTCAAGTTCTAGCAACGGTTTCC-3' (Ndel site in italics, stat codon underlined) and the reverse primer GFTpsBBam (SEQ ID NO:58) 5'-CGCGGATCCTCAGATGGTAACAGGGTCTCTGAGCACTGC-3' (BamHI site in italics, stop codon underlined) were used. In the same way, the forward primer GFTpsDNdel (SEQ ID NO:59) 5'-GCATGTTCCATATGTCGTCTGGAGAAACATTTCGTCC-3' and the reverse primer GFTpsDBam (SEQ ID NO:60) 5'-CGCGGATCCTCAAAATGGAACGTGGTCTCCTAG-3' were used for GFTpsD and the forward primer GFTpsENdel (SEQ ID NO:61) 5'-GCATGTTCCATATGTCTTTGGAAGTTTCAGCCTCTCCTG-3' and the reverse primer GFTpsEBam (SEQ ID NO:62) 5'-CGCGGATCCTCATATCGGCACAGGATTAATAAACAAAGAAGC-3' were used for GFTpsE.

The amplifications were performed using the Pfu DNA polymerase (Promega) in a final volume of 50 µl containing 5µl of Pfu DNA polymerase 10X buffer, 200 µM each dNTP, 0.4 µM each forward and reverse primer, 2.9 units Pfu DNA polymerase and 5 µl of 100-fold diluted cDNA (prepared as described above using the Marathon™ cDNA Amplification Kit (Clontech)). The thermal cycling conditions were as follows: 2 min at 95°C; 25 cycles of 30 sec at 95°C, 30 sec at 55°C and 4 min at 72°C; and 10 min at 72°C. The PCR product was purified on an agarose gel, eluted using the QIAquick® Gel Extraction Kit (Qiagen), digested with Ndel and BamHI and ligated into the similarly digested pET11a plasmid.

For construction of the GFTpsC-pET11 a expression vector, two separated PCR were employed to generate the insert flanked with the Ndel and BamHI cohesive ends. A first PCR was performed in the same condition as described above, using the forward primer GFTpsCpETFI (SEQ ID NO:63) 5'-TAATGGCACTTCAAGATTCAGAAGTTCCTC-3' and the reverse primer GFTpsCpETRI (SEQ ID NO:64) 5'-AAAAGGGAACAGGCTTCTCAAGCAATG-3' and a second PCR was performed using the forward primer GFTpsCpETF2 (shortened by AT at the 5'-end compared to GFTpsCpETFI) (SEQ ID NO:65) 5'-ATGGCACTTCAAGATTCAGAAGTTCCTC-3' and the reverse primer GFTpsCpETR2 (extended by GATC at the 5'-end compared to GFTpsCpETRI) (SEQ ID NO:66) 5'-GATCAAAAGGGAACAGGCTTCTCAAGCAATG-3'. The two PCR products were purified as described above, combined, denatured by 5 min boiling and cooled 5 min on ice. The resulting cDNA was used directly for ligation into the pET11a plasmid.

Ligation products were initially transformed into JM109 E. coli cells and the constructs were verified by restriction digestion and DNA sequencing.

### Sesquiterpene synthases expression.

For protein expression, the pET11 a plasmids containing the sesquiterpene synthase cDNAs as well as the empty pET11 a plasmid were transformed into the BL21(DE3) E. coli cells (Novagen). Single colonies were used to inoculate 5 ml LB medium. After 5 to 6 hours incubation at 37°C, the cultures were transferred to a 20°C incubator and left 1 h for equilibration. Expression of the protein was then induced by addition of 0.5 mM IPTG and the culture incubated over-night at 20°C.

The next day, the cells were collected by centrifugation, resuspended in 0.5 ml Extraction Buffer (50 mM MOPSO pH 7, 5 mM EDTA, 5 mM EDTA, 10% glycerol) and sonicated 3 times 30 s. The cell debris were sedimented by centrifugation 30 min at 18,000g and the supernatant containing the soluble proteins was recovered. The expression of the sesquiterpene synthases was evaluated by separation of the protein extract on a SDS-PAGE (SDS-polyacrylamid gel electrophoresis) and staining with coomassie blue and comparison to protein extract obtained from cells transformed with the empty plasmid.

A distinct band with the expected calculated molecular weight was observed for all constructs and the band was not present in the soluble proteins from *E. coli* transformed with the empty plasmid.

### Example 4 Enzyme Function Assay

The enzymatic assays were performed in sealed glass tubes using 50 to 100 µl of protein extract in a final volume of Extraction Buffer supplemented with 15 mM MgCl2 and 100 to 250 µM FPP (Sigma). The medium were overlaid with 1 ml pentane and the tubes incubated over-night at 30°C. The pentane phase, containing the sesquiterpenes, was recovered and the medium extract with a second volume of pentane. The combined pentane fractions were concentrated under nitrogen and analyzed by Gas Chromatography on a on a Hewlett-Packard 6890 Series GC system using a 0.25 mm inner diameter by 30 m SPB-1 (Supelco) capillary column. The carrier gas was He at constant flow of 1.6 ml/min. Injection was done at a split ratio of 2:1 with the injector temperature set at 200°C and the oven programmed from 80°C (0 min hold) at 7.5°C/min to 200°C (0 min hold) followed by 20°C/min to 280°C (2 min hold). Detection was made with a flame ionization detector. Compound identification was based on retention time identity with authentic standards when available. For confirmation of the products identities, samples were analyzed by combined capillary GC-MS using a Hewlett-Packard 6890 GC-quadrupole mass selective detector system, equipped with a 0.25 mm inner diameter by 30 m SPB-1 (Supelco) capillary column. The oven was programmed from 80°C (0 min hold) to 280°C at 7.5°C at a constant flow of 1.5 ml/min He. The spectra were recorded at 70eV with an electron multiplier voltage of 2200V.

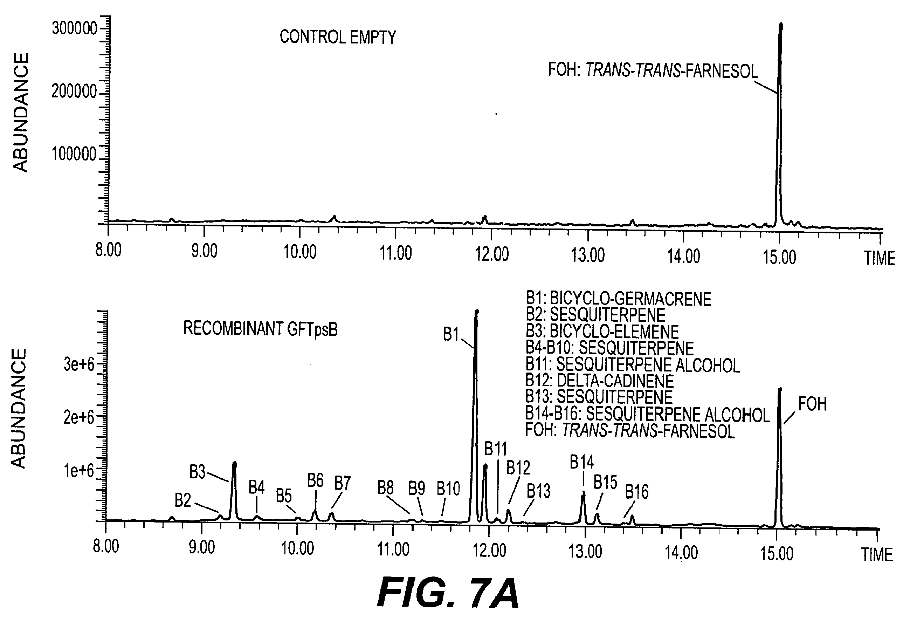

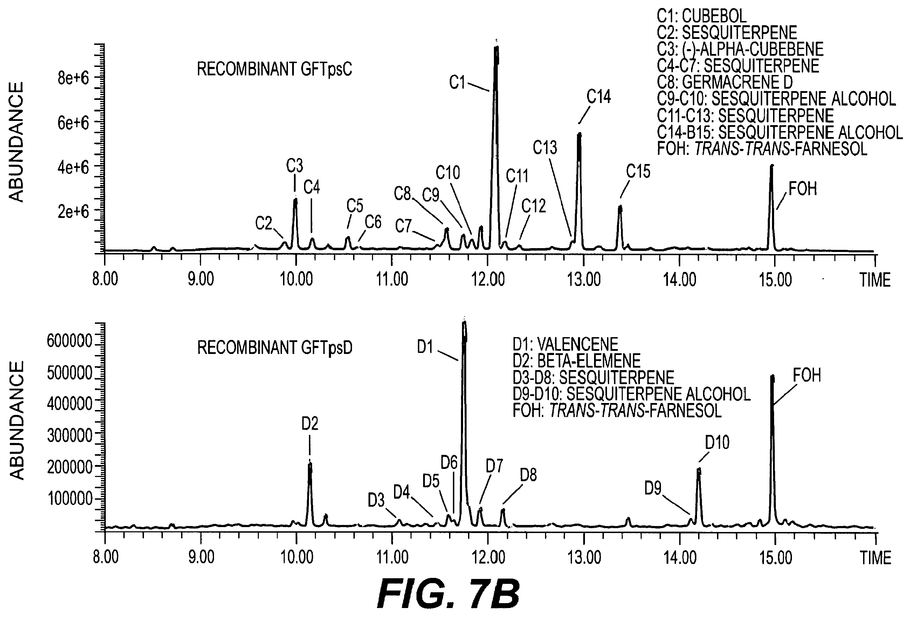

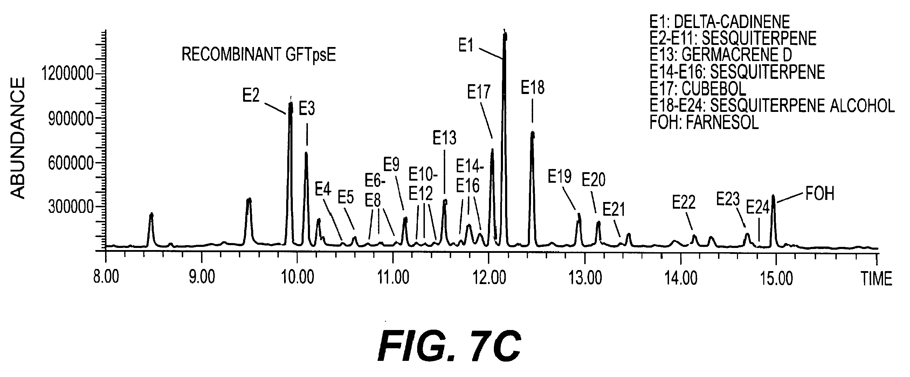

The enzyme activity of the different recombinant enzymes were evaluated in this assay using the soluble protein fraction and farnesyl-diphosphate as substrate. Sesquiterpene synthase activity was obtained for all clones tested and the product formed were characterized by retention time and GC-MS (Figure 7). The GFTpsB cDNA encoded a sesquiterpene synthase producing bicyclo-germacrene (Figure 3) as major product and at least 15 minor sesquiterpene olefins or oxygenated sesquiterpenes such as delta-cadinene (Figure 3) (byclo-elemene (Figure 3), resulting from heat rearrangement of bicyclo-germacrene was also observed in the GC trace). The GFTpsC cDNA encoded a multiple product forming sesquiterpene synthase with a major product being identified as cubebol (Figure 3). The enzyme also produced 3 other sesquiterpenes in a relative large proportion (-)- -cubebene and 2 oxygenated sesquiterpenes, and in small amounts, at least 11 sesquiterpene olefins olefins or oxygenated sesquiterpenes. GFtpsD encoded a sesquiterpene synthase that produces Valencene as a major product. Ten other minor peaks were also identified as sesquiterpene olefins or alcohols. Among them, β-elemene is a heat degradation product of germacrene A. GFTpsE encoded a sesquiterpene synthase producing a complex mixture of sesquiterpene compounds with δ-cadinene (figure 3) being slightly the most abundant. Cubebol and Germacrene D (Figure 3) were also identified in the mixture of products formed by GFTpsE.

The isolated sesquiterpene synthases demonstrated multiple product forming properties in the experiments described. For example, the cubebol synthase and the δ-cadinene produce 3 or 5 products in relatively large proportion. The bicyclogermacrene synthase produced a major sesquiterpene and several secondary products in trace amounts.

## Claims

1. An isolated nucleic acid selected from
(a) a nucleic acid comprising the nucleotide sequence substantially as set out in SEQ ID NO:9 or SEQ ID NO:10;
(b) a nucleic acid encoding the polypeptide substantially set out in SEQ ID NO:4 or SEQ ID NO:5; and
(c) a nucleic acid that hybridizes to the nucleic acid of (a) or (b) under low stringency conditions, wherein the polypetide encoded by said nucleic acid is a sesquiterpene synthase.

2. The isolated nucleic acid of claim 1, wherein the nucleic acid comprises the nucleotide sequence substantially as set out in SEQ ID NO:9 or SEQ ID NO:10.

3. The isolated nucleic acid of claim 2, wherein the nucleotide sequence is at least 85% identical to SEQ ID NO:9 or SEQ ID NO:10.

4. The isolated nucleic acid of claim 3, wherein the nucleotide sequence is at least 90% identical to SEQ ID NO:9 or SEQ ID NO:10.

5. The isolated nucleic acid of claim 4, wherein the nucleotide sequence is at least 95% identical to SEQ ID NO:9 or SEQ ID NO:10.

6. The isolated nucleic acid of claim 1, wherein the nucleic acid comprises the nucleotide sequence SEQ ID NO:9 or SEQ ID NO:10.

7. The isolated nucleic acid of claim 1, wherein the nucleic acid encodes the polypeptide substantially set out in SEQ ID NO:4 or SEQ ID NO:5.

8. The isolated nucleic acid of claim 7, wherein the polypeptide is at least 85% identical to SEQ ID NO:4 or SEQ ID NO:5.

9. The isolated nucleic acid of claim 8, wherein the polypeptide is at least 90% identical to SEQ ID NO:4 or SEQ ID NO:5.

10. The isolated nucleic acid of claim 9, wherein polypeptide is at least 95% identical to SEQ ID NO:4 or SEQ ID NO:5.

11. A polypeptide encoded by the nucleic acid of claim 1.

12. The isolated nucleic acid of claim 1, wherein the nucleic acid hybridizes to the nucleic acid of (a) or (b) under moderate stringency conditions.

13. The isolated nucleic acid of claim 14, wherein the nucleic acid hybridizes to the nucleic acid of (a) or (b) under high stringency conditions.

14. The isolated nucleic acid of claim 1, wherein the nucleic acid is isolated from a citrus selected from a grapefruit or an orange.

15. A vector comprising the nucleic acid of claim 1.

16. A host cell comprising the nucleic acid of claim 1.

17. An non-human organism modified to harbor the nucleic acid of claim 1.

18. The non-human organism of claim 17, wherein said organism is a plant or microorganism.

19. A method of making a recombinant host cell comprising introducing the vector of claim 15 into a host cell.

20. A method of producing a polypeptide comprising culturing the host cell of claim 16 under conditions to produce said polypeptide.

21. An isolated polypeptide comprising an amino acid sequence substantially as set out in SEQ ID NO:4 or SEQ ID NO:5.

22. The polypeptide of claim 21, wherein the amino acid sequence is at least 85% identical to SEQ ID NO:4 or SEQ ID NO:5.

23. The polypeptide of claim 22, wherein the amino acid sequence is at least 90% identical to SEQ ID NO:4 or SEQ ID NO:5.

24. The polypeptide of claim 23, wherein the amino acid sequence is at least 95% identical to SEQ ID NO:4 or SEQ ID NO:5.

25. A method of making at least one sesquiterpene synthase comprising
culturing a host modified to contain at least one nucleic acid sequence under conditions conducive to the production of said at least one sesquiterpene synthase
wherein said at least one nucleic acid is chosen from
(a) a nucleic acid comprising the nucleotide sequence substantially as set out in SEQ ID NO:9, or SEQ ID NO:10;
(b) a nucleic acid encoding the polypeptide substantially set out in SEQ ID NO:4, or SEQ ID NO:5; and
(c) a nucleic acid that hybridizes to the nucleic acid of (a) or (b) under low stringency conditions, wherein the polypetide encoded by said nucleic acid is a sesquiterpene synthase.

26. The method of claim 25, wherein the nucleic acid comprises the nucleotide sequence substantially as set out in SEQ ID NO:9, or SEQ ID NO:10.

27. The method of claim 25, wherein the nucleic acid encodes the polypeptide substantially set out in SEQ ID NO:4, or SEQ ID NO:5.

28. The method of claim 25, wherein said host is a plant or microorganism chosen from bacterial cells, yeast cells, plant cells, and animal cells.

29. A method of making at least one terpenoid comprising
A) contacting at least one acyclic pyrophosphate terpene precursor with at least one polypeptide encoded by a nucleic acid chosen from
(a) a nucleic acid comprising the nucleotide sequence substantially as set out in SEQ ID NO:9, or SEQ ID NO:10;
(b) a nucleic acid encoding the polypeptide substantially set out in SEQ ID NO:4, or SEQ ID NO:5; and
(c) a nucleic acid that hybridizes to the nucleic acid of (a) or (b) under low stringency conditions, wherein the polypetide encoded by said nucleic acid is a sesquiterpene synthase.
B) isolating at least one terpenoid produced in A).

30. The method of claim 29, wherein said at least one terpenoid is chosen from sesquiterpenes.

31. The method of claim 29, wherein said at least one acyclic pyrophosphate terpene precursor is farnesyl-pyrophosphate.

32. The method of claim 31, wherein said at least one sesquiterpene is valencene.

33. The method of claim 29, wherein the nucleic acid comprises the nucleotide sequence substantially as set out in SEQ ID NO:9, or SEQ ID NO:10.

34. The method of claim 29, wherein the pH at the time of contacting is 7 or less.
